# EUROPEAN PATENT APPLICATION

(11) **EP 1 715 344 A1**
(43) Date of publication of application: **25.10.2006**
(21) Application number: 05075967.9
(22) Date of filing: 22.04.2005
(51) Int. Cl.: G01N 33/569, G01N 33/543

(54) **Immobilisation of antigenic carbohydrates to support detection of pathogenic micro-organisms**

(71) Applicant: Universiteit Utrecht Holding B.V., 3584 CM Utrecht (NL)
(72) Inventor: The designation of the inventor has not yet been filed
(74) Representative: Winckels, Johannes Hubertus F.

(57) **Abstract**

The invention relates to the field of chemistry and diagnosis, more in particular to diagnosis of current and/or past and/or symptomless infections or of a history of exposure to a gram-negative-bacterium (such as an enterobacteriaceae or a legionella). Even more in particular, the invention relates to the screening of animals or animal products for the presence of unwanted/undesired microorganisms. The invention further relates to a method for screening samples for the presence of antibodies directed against unwanted/undesired microorganisms and preferably such a method is performed with help of a biosensor. The invention also relates to a method for immobilising polysaccharides to solid surfaces. The invention furthermore provides solid surfaces with immobilised polysaccharides as well as applications of such surfaces.

## Description

The invention relates to the field of chemistry and diagnosis, more in particular to diagnosis of current and/or past and/or symptomless infections or of a history of exposure to a gram-negative-bacterium (such as an enterobacteriaceae or a legionella). Even more in particular, the invention relates to the screening of animals or animal products for the presence of unwanted/undesired microorganisms. The invention further relates to a method for screening samples for the presence of antibodies directed against unwanted/undesired microorganisms and preferably such a method is performed with help of a biosensor. The invention also relates to a method for immobilising polysaccharides to solid surfaces. The invention furthermore provides solid surfaces with immobilised polysaccharides as well as applications of such surfaces.

The world is full of gram-negative bacteria, many of which are members of the family Enterobacteriaceae. Members of this family are found in the gastrointestinal tract of animals, but many are also free living in soil and water. Members of the family Enterobacteriaceae have very complex antigenic structures. Moreover, they comprise multiple antigens that are identified as K antigens, H antigens and O antigens. The K antigen is the acidic polysaccharide capsule. The capsule has many functions including evasion from the immune system of the infected host and adhesion to the epithelium of the host. The H antigen is located on the flagella.

The outer portion of the cell wall in gram-negative bacteria is chiefly composed of lipopolysaccharides (LPS). LPS is composed of lipid A which is buried in the outer membrane, a short carbohydrate core and optionally a chain of polysaccharides that is made up of repeating units. The O-antigens are located on the polysaccharide. Lipid A is the toxic constituent of the LPS. As cells lyse, LPS is released, leading to fever and complement consumption. It also interferes with coagulation and at high concentrations eventually leads to a state of shock.

As a non-limiting example one member of the enterobacteriaceae, salmonella, will be discussed in more detail. A large number of the subspecies of the genera of *Salmonella enterica* are important pathogenenic bacteria for humans and animals. Besides that animals go into a pathological episode, animals can be symptomless carriers of the bacteria. Contaminated animals can be a source of these pathogens threatening public health for example through the food that these animals produce. As many stakeholders consider the number of food-borne salmonella infections unacceptable, measures have to be taken to contain this pathogen in the food chain.

Salmonella is of major significance as a pathogenic microorganism in food-borne infections in humans, causing mild to severe clinical effects. In The Netherlands, 5% of all identified cases of gastroenteritis is salmonellosis (Edel et al., 1993; Hoogenboom Verdegaal et al., 1994). The average incidence of this infection is 450 cases per 100,000 person years at risk, which is similar to that in other industrialized countries (Berends et al., 1998). Despite the 2480 serotypes identified in the group of *S. enterica* up to 2001 [Popoff, 2001), only a small number have been involved in human infections (Grimont et al., 2000). *Salmonella typhimurium* plus *Salmonella enteritidis* represented >75% of all salmonella isolates from human sources sent to the Dutch National Salmonella Centre at the RIVM in 2002 (Van Pelt et al., 2003). This percentage consisted of 51% contributed by contact with chicken products (poultry 15%; eggs 36%)(Van Pelt et al., 2003).

Detection of immunoglobulins in the body fluids of organisms (serology) is a way to establish a history of exposure of animals and humans to infectious agents. A humoral response against salmonella antigens can be detected in chickens 1 week post-infection and persists for at least 10 weeks even if the bird is no longer culture-positive (Holt, 2000). The antigenic determinants of salmonella are, as described above, composed of somatic (O), flagellar (H) and surface (Vi) antigens (Holt, 2000). Variations in the composition of antigens correlate with different salmonella serotypes.

Typically, serology is faster than culture-typing of the disease-causative organism. Fast and specific detection of potential salmonella-positive herds and flocks is of importance in order to take adequate measures in production processes. The detection of antibodies in serum and blood samples from food-producing animals reporting the presence of zoonotic pathogens is therefore of significance. Such information is then used as the input for risk-assessment and rational slaughtering of potentially pathogen-contaminated animals in order to be able to increase food safety, but also to improve occupational hazards and to reduce spreading of the pathogens in the environment.

A number of serological tests have been developed for the detection of invasive salmonella species. Among many such methods, agglutination and ELISA have most commonly been used (Barrow, 2000). Agglutination tests have been used successfully to eradicate *Salmonella pullorum* from poultry flocks. However, the approach is cumbersome, laborious and not suitable for large-scale screening programs according to modern standards. Several ELISA procedures, which are considered relatively cheap and fast, have therefore been developed to detect anti-*S*. *enteritidis* and *S. typhimurium* antigen responses in poultry sera (Barrow et al., 1996; Thorns et al., 1996; de Vries et al., 1998; Barrow, 2000; Yamane et al., 2000).

The use of biosensors also promises to be useful, cheap and rapid in this area of analysis. In addition, the technique is able to detect multiple analytes of any biomolecular type in a single run. A biosensor is defined as an analytical device consisting of (i) a re-usable immobilized biological ligand that 'senses' the analyte, and (ii) a physical transducer, which translates this phenomenon into an electronic signal.

The surface plasmon resonance (SPR) phenomenon was first recognized in the early 1960s (Kretschmann and Raether, 1968) and the first SPR biosensors were introduced in the 1980s (Liedberg et al., 1983). It took until the late 1980s and early 1990s before the first commercially available SPR-based biosensor equipment was released on the market. Initially, this type of biosensor attracted the interest of pharmaceutical companies as a secondary tool for both selective and sensitive *in vitro* screening of promising novel pharmaceutical products from combinatorial libraries. It proved to be a valuable alternative for classic approaches such as ELISA procedures. Moreover, it offers real-time measurement of the binding event in contrast to end-point determinations. The benefits of this analytical approach have also been recognized by many other life science disciplines, including food sciences (Ivnitski et al., 1999; Medina, 1997). So far, only a few publications on SPR biosensing have addressed the detection of pathogenic microorganisms, for example the use of immobilized *Escherichia coli* O157:H7 cells to screen the performance of anti- E. coli O157:H7 antibodies (Medina et al., 1997), and the use of these antibodies to detect E. coli O157:H7 cells (Fratamico et al., 1997).

In Jongerius-Gortemaker et al. (2002) a study to the suitability of an SPR optical biosensor to detect antibodies in serum and blood indicating a humoral reaction to invasion with *Salmonella* serotypes *enteritidis* and *typhimurium* was initiated. In this study, use was made of immobilised flagellar antigen fusion proteins. After thorough analysis it is concluded that the sensitivity and/or the robustness of this system was not sufficient and in particular not for high-throughput screening of for example poultry at the slaughter line in an abattoir, processing animals at the rate of several thousands per hour.

The goal of the present invention is to provide for a method that has an improved sensitivity and/or an improved robustness. This goal has been reached by developing a carrier with immobilised somatic or so-called O-antigens. As described, the O-antigens are located on the lipopolysaccharides and the composition of the polysaccharide varies and corresponds with the serovar of the salmonella (sub)species. Every serotype can, amongst others, be described by a number of O-antigens and are typically coded with a number, such as 04, 06 or 012. The O-antigens can be found as repeating units on the polysaccharide part of the LPS. The length of the polysaccharide also varies and can be between zero (rough LPS) and more than 50 repeating units (smooth LPS).

Within the *Salmonella enterica* family, different serogroups can be distinguished; each group comprises at least one specific O-antigen. The salmonella serovars of importance in chicken and pigs are listed with their O-antigen profile in Table 1. In Denmark, Germany, Greece and The Netherlands, 39.5% of all salmonella-positive pigs sampled at the abattoir were determined as *S. typhimurium*. Dependent of country, other important isolates from pigs were *S. derby* (17.1%), *S. infantis* (8.0%), *S. panama* (5.1%), *S. ohio* (4.9%), *S. london* (4.4%), *S. livingstone* (3.1%), *S. virchow* (2.7%), *S. bredeny* (2.1%), *S. mbandaka* (1.1%), *S. Brandenburg* (1.0%), *S. goldcoast* (0.8%).

In case of chickens, 14% of the chickens were salmonella-positive at flock level in 2002 in The Netherlands. The predominant serovar was in that case *S. paratyphi B var. java.* At the retail level a comparable percentage (13.4%) was found in the Netherlands. The most frequent salmonella serovars isolated from broilers in 14 EU member states were *S. paratyphi B var. java* (24.7%), *S. enteritidis* (13.6%), *S. infantis* (8.0%), *S. virchow* (6.7%), *S. livingstone* (5.7%), *S. mbandaka* (5.5%), *S. typhimurium* (5.3%), *S. senftenberg* (5.0%), *S. hadar* (3.7%). *S. paratyphi B var. java* is dominating, but this is fully attributable to The Netherlands.

**Table 1. Some salmonella serovars considered as important zoonotic agents in broilers and in pigs listed with their O-antigen profiles (Popoff, 2001)**

| **Salmonella serovar** | **Chicken (C)/ pigs (P)** | **O-antigen profile** | **serogroup** |
|---|---|---|---|
| *Brandenburg* | P | 4, [5], 12 | B |
| *Bredeny* | P | 1, 4, 12, 27 | B |
| *Derby* | P | 1^{a}, 4, [5]^{b}, 12 | B |
| *Enteritidis* | C | 1, 9, 12 | D₁ |
| *Goldcoast* | C/P | 6, 8 | C₂ |
| *Infantis* | C/P | 6, 7, 14 | C₁ |
| *Livingstone* | P | 6, 7, 14 | C₁ |
| *London* | P | 3, 10, [15] | E₁ |
| *Mbandaka* | P | 6, 7, 14 | C₁ |
| | | 3, 10, [15], [15, | E₁ |
| *Meleagridis* | P | 34]^{c} | |
| *Ohio* | P | 6, 7, 14 | C₁ |
| *Panama* | P' | 1, 9, 12 | D₁ |
| *Paratyphi B var.* | C | 1, 4, [5], 12 | B |
| *Java* | | | |
| *Typhimurium* | C/P | 1, 4, [5], 12 | B |
| *Virchow* | P | 6, 7, 14 | C₁ |

| | | | |
|---|---|---|---|
| ^{a} O antigen determined by phage conversion is indicated by underlining ^{b} O antigens which may be present or absent are indicated in square brackets ^{c} lysogenized by phage ε₁₅ [15] and by phage ε₃₄ [15, 34] | | | |

In a first embodiment, the invention provides a method for immobilisation of a polysaccharide on a carrier, comprising contacting said polysaccharide with an oxidising agent and a protein to obtain a polysaccharide-protein complex and coupling said polysaccharide-protein complex to said carrier. The sensitivity of such a prepared carrier is only acceptable when the lipopolysaccharide (O-antigen) before the immobilisation on the carrier is oxidised in the presence of a protein. Although we do not wish to be bound by any theory, it is currently thought that the aldehyde groups that result from the oxidation of the polysaccharides are capable of reacting with the amino groups of the protein to form a substituted imine (Schiff-base binding). Upon injection over (an activated) carrier (for example a sensorchip) the available aldehyde groups react with hydrazide to form hydrazon. The following reduction stabilises not only the covalent binding between the carrier (for example a carrier comprising dextran) and the polysaccharide but also the imine binding between protein and polysaccharide. As will be explained in more detail in the experimental part, polysaccharides (O antigens) of different salmonella sera types have been immobilised on a carrier. The prepared carriers were subsequently subjected to an SPR-analysis with standard sera. The obtained serological response was used as an indicator for success of the method. When coupling reactions were performed without the oxidation step no response of reference sera could be detected.

Preferably, the immobilisation/coupling of the polysaccharide-protein complex to a carrier is such that high sensitivity and/or robustness is obtained. Whereas flagellar antigens denature and lose their antigenicicty towards serum antibodies while the sensor chip has to be regenerated for a next analysis cycle with relatively harsh solvents, the somatic antigens are found rather stable towards these regeneration solvents. In fact, the loss of immobilized O-antigen activity is believed to be primarily associated with degradation of the solid surface, namely gradual loss of dextran layer attached to the goldfilm, to which the antigens are bound. The method according to the invention results in a carrier that is more robust compared to a carrier of the prior art.

Preferably, the invention provides a method for immobilisation of a polysaccharide on a carrier, comprising contacting said polysaccharide with an oxidising agent and a protein to obtain a polysaccharide-protein complex and coupling said polysaccharide-protein complex to said carrier, wherein said polysaccharide is derived from a gram-negative bacterium and even more preferably wherein said polysaccharide is derived from an enterobacteriaceae. Yet even more preferably, said polysaccharide is derived from a gram-negative bacterium that is a human or veterinary or plant pathogen. Examples of such polysaccharides are polysaccharides derived from a salmonella (sub)species. Other examples are polysaccharides derived from *Eschericia coli* species (for example E.coli 0157) and the bacterial species outlined in Table 2.

**Table 2. Examples of LPS-containing bacteria pathogenic to human and/or animals.**

| Bacterial species | Mainly found in | affecting |
|---|---|---|
| *Campylobacter coli* | Swine | Humans |
| *Campylobacter jejuni* | Avian species, dogs | Humans |
| *Campylobacter lari* | Seagull | Humans |
| *Escherichia coli O*157 | Ruminants | Humans |
| *Legionella pneumophila* | Water | Humans |
| *Listeria monocytogenes* | As food process-contaminant | Humans |
| *Salmonella choleraesuis* | Swine | Swine |
| *Salmonella enteritidis* | Avian species, swine | Humans |
| *Salmonella gallinarum* | Avian species | Chickens |
| *Salmonella goldcoast* | Swine | Humans |
| *Salmonella infantis* | Chickens | Humans |
| *Salmonella livingstone* | Swine | Humans |
| *Salmonella meleagridis* | Swine | Humans |
| *Salmonella pollorum* | Avian species | Chickens |
| *Salmonella typhimurium* | Avian | Humans, horses |
| *Streptococcus suis* | Swine | Swine, humans |
| *Vibrio cholerae* (non-O1) | Aquatic animals | Humans |
| *Vibrio paraheamolyticus* | Aquatic animals | Humans |
| *Vibrio vulnificus* | Aquatic animals | Humans |
| *Yersinia enterocolitica* | Swine | Humans |

As will be explained in more detail later, a carrier comprising an immobilised polysaccharide (O-antigen) is particularly useful in the diagnosis of the mentioned LPS-containing bacteria.

The term "polysaccharide" is intended to mean an entity comprising two or more glycoside linked monosaccharide units and embraces, amongst others, an oligosaccharide (2-10 residues) as well as a polysaccharide (more than 10 monosaccharides). The linking may result in linear or branched polysaccharide. In a preferred embodiment, the invention provides a method for immobilisation of a polysaccharide on a carrier, comprising contacting said polysaccharide with an oxidising agent and a protein to obtain a polysaccharide-protein complex and coupling said polysaccharide-protein complex to said carrier, wherein said polysaccharide is a lipopolysaccharide (LPS), i.e. a polysaccharide comprising lipid A. It is clear to a skilled person that the used (lipo)polysaccharide must comprise at least one antigenic structure and one group available/suitable for providing a linkage between the protein and the polysaccharide. More details in respect of the last item will be provided later on. Hence, as long as the (lipo)polysaccharide comprises an antigenic structure and a group suitable for providing a linkage between the protein and the polysaccharide an immobilization method of the invention may be used to obtained a sensitive and/or robust carrier.

The LPS is expressed at the cellular exterior and is part of the bacterial cellular wall. The expression of LPS is not under direct genetic control, so that LPS is a pool of different molecules with varying composition of the lipid A part in terms of the attached aliphatic chain. Moreover, the bacterial cell may synthesize rough LPS with no or a short carbohydrate chain, or smooth LPS with a mature carbohydrate chain existing of more than 50 repeating units expressing its antigenicity. In addition to this heterogeneity, within a single molecule LPS, several O-antigen entities, which are distinctively numbered, may be expressed. An O-antigen profile is, however, per definition unique for a salmonella serogroup. A complete serotyping of a salmonella also includes the H-antigens as well as the Vi-antigens.

LPS may be obtained by a variety of methods and the experimental part describes in more detail the use of a trichloric acid extraction (optionally followed by ethanol extraction and dialysis) according to Staub (1965) for this purpose. Other examples of suitable extraction methods are described by Wilkons (1996) and include, but are not restricted to, extractions with diethylene glycol, dimethyl sulphoxide, NaCl-diethyl ether (1:2 (v/v)), NaCl-butan-1-ol (1:1 (v/v)), aqueous EDTA, NaCl-sodium citrate, aqueous phenol or aqueous phenolchloroform petroleum.

The purity of the obtained/used LPS batch is considered not to be extremely critical. It is experienced that the LPS does not have to be completely free of contaminants. The specific coupling reaction provides a certain degree of selectivity. Moreover, as described in the experimental part, the used/obtained LPS (preferably an LPS batch) is optimised in respect of the amount of protein necessary for an optimal response. It is clear to a skilled person that the LPS preferably comprises not much rough LPS. The preferred LPS batch essentially comprises smooth LPS.

Although we do not wish to be bound by any theory it is currently thought that the presence of a 2-keto-3-deoxy-octonic acid (KDO) and/or a glycerol-mannoheptose (Hep) and/or a GlcNAc in the core of the LPS molecule is needed for a covalent coupling.

Although a lot of different bacteria are employed by the term gram-negative bacteria it is believed that LPS from all these bacteria are suitable for use in the presently claimed invention as long as the LPS comprises at least one constituent with non-conjugated or de-conjugated vicinal hydroxy groups, preferably in the core region of the LPS molecule. In salmonella, most likely candidate constituents are KDO and Hep and GlcNAc residues. The presence or absence of such a KDO and/or Hep and/or GlcNAc group is indirectly genetically determined. Although the genetic information necessary to construct the species-, serotype or even strain specific monosaccharides is present in the corresponding organism, it depends on the growth circumstances whether said LPS contains aldehyde-convertible monosaccharides in the core region.

There are of course also other sources of LPS available, such as buying it commercially.

In a preferred embodiment, the invention provides a method for immobilisation of a polysaccharide on a carrier, comprising contacting said polysaccharide with an oxidising agent and a protein to obtain a polysaccharide-protein complex and coupling said polysaccharide-protein complex to said carrier, wherein said protein is a protein (for example a serum protein) with a certain amount of (primary) amines. Preferably, at least some of these amines are not sterical hindered and/or are not participating in non-covalent bindings, such as H-H bridges or dipole-dipole interactions and/or are not protonated. Such a protein preferably does not have or hardly have, any immunogenic properties and hence cross-reacting antibodies directed to the used protein are avoided as much as possible. Examples of suitable proteins are haemoglobin (Hb), ovalbumin (Ob), myoglobin (Mb) and serum albumin (SA). The biosensor response of different standard sera on immobilised LPS oxidized in the presence of Hb or Ob or Mb or SA were determined. Serum albumin, myoglobin and haemoglobin gave the most promising results. In a preferred embodiment the protein is haemoglobin or myoglobin.

The necessary protein is obtained commercially or by (overexpressing) in a suitable expression system or by isolating it from a suitable source. Haemoglobin has for example been obtained by isolating it from blood. Preferably the used protein batches are as pure as possible, thereby circumventing as much cross-reactions as possible. It is however experienced that small amounts of contamination are allowed without jeopardising the sensitivity and/or robustness of the obtained carriers.

The ratio (lipo)polysachcharide versus protein depends, amongst others, on the used protein. Experiments with Hb have shown that concentrations between 15 and 50 % (m/m) have resulted in satisfactory results. When bovine serum albumin is used much lower ratios, between 0.7 and 7% (m/m), are used. Some examples: the optimal Hb concentration for *S. livingstone* LPS is around 50% (m/m) and for *S. enteritidis* LPS the optimal Hb concentration is 15% (m/m).

The isolated LPS preparations are oxidised in the presence of a protein facilitated by an oxidising agent. In a preferred embodiment the invention provides a method for immobilisation of a polysaccharide on a carrier, comprising contacting said polysaccharide with an oxidising agent and a protein to obtain a polysaccharide-protein complex and coupling said polysaccharide-protein complex to said carrier, wherein said oxidising agent is capable of oxidising vicinal diols.

Even more preferably, the oxidising agent exclusively oxidises vicinal diols, for which (sodium)periodate is a suitable oxidant.

Again we do not wish to be bound by any theory but it is currently thought that periodate will induce an oxidative disruption of linkages between vicinal diols on especially carbohydrate moieties, as in e.g. mannose, to yield aldehyde functionalities. This reaction is typically performed in buffers at a pH range between 4.5 and 5.5 in the dark using a (preferably) freshly prepared 1-100 mM sodium meta-periodate in 0.1 M sodium acetate. The oxidation is performed in the presence of a protein in the ranges as discussed above. The bis-aldehyde compounds, like the oxidised monosaccharide constituents in the polysaccharide chain of LPS, may react with any amino group in a protein and may form a Schiff-base linkage resulting in a substituted imine. When one or both of the vicinal hydroxyl groups is condensed in a covalent sugar linkage, the hydroxyl function is lost and no oxidation occurs. This is the case in many branched and/or linearly linked oligo- and polysaccharides. In the case of salmonella LPS, the inner core structure carries in most cases an oxidisable Gal, GlcNAc, Hep and/or KDO, but non-reducing Hep and KDO constituents are most susceptible for oxidation, in particular at very mild oxidation conditions at concentrations less than 6 mM meta periodate. Because the core region is a rather conserved part of LPS from different Enterobactericeae, (lipo)polysaccharides of members of the Enterobactericeae may be applied in a method of the invention.

Periodate will also oxidise, when present, certain aminoethanol derivatives such as the hydroxylysine residues in collagen, as well as methionine (to its sulfoxide) and certain thiols (usually to disulfides). In addition, N-terminal serine and threonine residues of peptides and proteins can be selectively oxidized by periodate to aldehyde groups. These reactions, however, usually occur at a slower rate than oxidation of vicinal diols and the presence of such group does not substantially interfere with a method according to the invention.

The invention also provides a method for immobilisation of a polysaccharide on a carrier, comprising contacting said polysaccharide with an oxidising agent and a protein to obtain a polysaccharide-protein complex and coupling said polysaccharide-protein complex to said carrier, further comprising a step which results in ending/stopping the oxidation process, for example by desalting of said polysaccharide-protein complex. This is for example accomplished with help of a NAP-5 column. However the person skilled in the art is aware that many other methods exist which have the same effect, for example adding a reductor or an easily oxidisable molecule such as glycerol. Preferably, the way of stopping the oxidation is such that at the same time a buffer change is accomplished, for example HPLC, FPLC, dialysis, ion-exchangers, gel electrophoresis or ultrafiltration.

For storage purposes, the production of evaporated aliquots, after addition of protein, is also described within the experimental part. This results in the presence of a large stock of reproducible material.

The invention therefore further comprises the obtained intermediate, i.e. the preparation of in the presence of protein oxidised polysaccharide, optionally desalted and optionally evaporated.

Preferably, the used carrier is made of an inert, non-hydrophobic material and the binding of the LPS-protein complex to said carrier is covalent. Even more preferred such a carrier has a low protein binding or low biomolecular binding. Examples are a carrier of glass or silica or of a non-hydrophobic plastic.

In a preferred embodiment the invention provides a method for immobilisation of a polysaccharide on a carrier, comprising contacting said polysaccharide with an oxidising agent and a protein to obtain a polysaccharide-protein complex and coupling said polysaccharide-protein complex to said carrier, further comprising activating the surface of said carrier. In an even more preferred embodiment, said carrier comprises a glass surface coated with gold and even more preferred said carrier is modified with a carboxymethylated dextran layer wherein said dextran layer is activated with 1-ethyl-3-(3-dimethylaminopropyl)carbodiimide hydrochloride and N-hydroxysuccinimide. The activation is followed by preparation with carbohydrazine. In the next step the polysaccharide-protein complex is added to the activated dextran layer. The reactive aldehyde functionalities react spontaneously with the hydrazide to hydrazones, which are then reduced to stabilise the covalent bonds.

Prior to routinely use, the performance of chip-conjugated LPS to bind anti-Enterobacterium (for example salmonella) antibodies is assessed using reference polyclonal agglutination sera.

Depending on the analytical/diagnostic question asked it is decided whether one or for example at least two different serogroup-representing carbohydrates, preferably 4 different serogroup-representing carbohydrates are used. In case one is interested in knowing which particular serogroup is present, multiple (the amount of which is different on the particular question asked and on the used apparatus) different serogroup-representing carbohydrates are used and if one just wants to know whether for example an animal is or has been infected by a particular serogroup, a single serogroup-representing carbohydrate may be oxidised in the presence of a protein and immobilised on a carrier. The use of at least two different serogroup-representing carbohydrates results in a carrier that can be used in a multi-serogroup analysis. More preferably at least three and even more preferred at least more than three (for example four) different polysaccharides are used. These polysaccharides may be oxidised in the presence of one type of protein or in the presence of different types of protein. The skilled person is capable of making any sensible combination. For example, to be able to detect more than 90% of all salmonella infections serogroups B, C and D in chicken and serogroups B, C, D and E in pigs should be represented.

Using one type of serogroup-representing carbohydrates is extremely useful if one is interested in the question whether or not a certain type of bacterium is or was present. Using multiple different serogroup-representing carbohydrates is for example useful if one wants to determine whether an animal is or was infected by any gram-negative bacteria (for example enterobacteriaceae).

In a preferred embodiment the invention provides a method for immobilisation of a polysaccharide on a carrier, comprising contacting said polysaccharide with an oxidising agent and a protein to obtain a polysaccharide-protein complex and coupling said polysaccharide-protein complex to said carrier, wherein said carrier is a biosensor chip. Such a biosensor chip is commercially available (for example that produced by Biacore) and hence no further information will be provided.

In another embodiment the invention provides a carrier obtained by the method according as described above or a carrier comprising an immobilised polysaccharide-protein complex on its surface. In yet another embodiment the invention provides biosensor comprising a carrier according to the invention. Whether the carrier is obtained by a method according to the invention can for example be determined by extracting the polysaccharides from said carrier and determining whether covalently linked protein is present. As already discussed above the carrier may also comprise different immobilised polysaccharides (for example O-antigens) possibly in combinations with different types of protein. However, also one type of protein may be used in the oxidation of different polysaccharides.

Whether a carrier and/or chip of the invention is employed can for example be determined with help of MALDI-MS possibly in combination with proteolytic digestion. Such an analysis provides information with respect to the used protein and polysaccharide. With help of acidic hydrolysis the polysaccharide-protein complexes are released from the carrier. Such an obtained mixture is then subjected to LC-MS/MS analysis before and after proteolytic hydrolysis. The obtained complex may also be subjected to a monosaccharide analysis, for example GC-MS following methanolysis and/or Smith degradation, from which it is determined which type of LPS is used. This information is furthermore used to determine whether KDO, Hep or other sugars have been oxidised.

A carrier of the invention may be used in different detection systems, for example optical, thermal, acoustic, amperometric, magnetic or chemical and a carrier of the invention may be used in any biomolecular interaction assay (BIA) or any affinity assay (AA). As a non-limiting example, the use of optical detection via Surface Plasmon Resonance is described in more detail.

The invention provides a Surface Plasmon Resonance detection system comprising a biosensor as described above. The gold layer in the sensor chip creates the physical conditions required for Surface Plasmon Resonance (SPR). The principle of SPR will be described in the context of Biacore instruments. They incorporate the SPR phenomenon to monitor biomolecular interactions in 'real-time'. At an interface between two transparent media of different refractive index such as glass and water, light coming from the side of higher refractive index is partly reflected and partly refracted. Above a certain critical angle of incidence no light is refracted across the interface and total internal reflection (TIR) occurs at the metal film-liquid interface. This is where light travels through an optically dense medium such as glass, and is reflected back through that medium at the interface with a less optically dense medium such as buffer. Although the incident light is totally reflected, the electromagnetic field component, termed the evanescent wave, penetrates a distance on the order of one wavelength into the less optically dense medium. The evanescent wave is generated at the interface between a glass prism (high refractive index) and a layer of buffer (lower refractive index). If the interface between the media of higher and lower refractive indices is coated with a thin metal film (a fraction of the light wavelength), then the propagation of the evanescent wave will interact with the electrons on the metal layer. Metals contain electron clouds at their surface, which can couple with incident light at certain angles. These electrons are also known as plasmons, and the passage of the evanescent wave through the metal layer causes the plasmons to resonate, forming a quantum mechanical wave known as a surface plasmon. Therefore, when surface plasmon resonance occurs, energy from the incident light is lost to the metal film resulting in a decrease in the reflected light intensity. The resonance phenomenon only occurs at an acutely defined angle of the incident light. This angle is dependent on the refractive index of the medium close to the metal-film surface. Changes in the refractive index of the buffer solution (e.g. an increase in surface concentration of solutes), to a distance of about 300 nm from the metal film surface will therefore alter the resonance angle. Continuous monitoring of this resonance angle allows the quantitation of changes in refractive index of the buffer solution close to the metal-film surface. In 'real-time' Biacore, the metal film properties, wavelength, and refractive index of the glass (denser medium) are all kept constant, and as a result SPR can be used to monitor the refractive index of the aqueous layer immediately adjacent to the metal (gold) layer. In the Biacore system the chip is composed of glass, has 4 channels and the associated gold layer is covered with a layer of dextran chemically modified to facilitate immobilisation of ligands such as antibodies or antigens. Any changes in mass that occur due to binding of the analyte with the immobilised antibody on the sensor chip will cause a change in SPR angle, which is monitored in 'real-time' and quantified as a sensorgram. A mass change of approximately 1 kRU (1,000 RU) corresponds to a mass change in surface protein concentration of 1 ng/mm². Typical responses for surface binding of proteins are of the order of 0.1-20 kRU.

There is no need to label molecules with fluorescent or radioactive tags- so avoiding the possibility that labels may compromise activity and moreover no difficult or expensive chemistry is necessary for labelling.

The obtained carriers can be used in different types of analysis, such as bacteriology (direct assay) or serology (indirect assay).

An example of a serological assay is a method for determining the presence of an antibody directed to an antigen of a gram-negative bacteria in a sample, comprising contacting said sample with a carrier or a biosensor as described above and determining whether the carrier has bound any antibody. Such a method is for example very suitable for determining the presence of an antibody directed against an O antigen and thus it is indirectly established whether an infection is present or whether a recent infection has occurred. Such a method is for example used to screen slaughter animals for salmonella or to screen animals for salmonella before they are exported abroad. Moreover, the method is also applied to samples obtained from living (for example, farm or zoo) animals.

Examples of samples that can be used in such a method are tissue sample, body fluid, secretes or excretes and more detailed examples are blood, blood derived samples, tissue, meat juice, milk, egg, fluids from an eye, saliva or faeces. As already outlined the samples can be obtained from dead as well as living animals.

A method according to the invention is not limited to a certain immunoglobulin (sub)type but can in principle be every (iso)type immunoglobulin such as (s)IgA₁, (s)IgA₂, IgD, IgG₁, IgG₂, IgG₃, IgG₄, IgM, IgY. Moreover, it may also be any other antigen-binding material. Preferably, such an antigen-binding material is a biomarker of a (history) of infection.

Such a serological assay is for example directed to one particular serogroup-representing carbohydrate or to different (i.e. multi analyte) serogroup-representing carbohydrates and hence such a method is for example used to determine the presence or absence of a certain salmonella (sub)type.

An example of a bacteriological assay is a method for determining the presence of a gram-negative bacterium in a sample, comprising contacting said sample with a predetermined amount of antibodies directed against an antigen of said bacterium and determining the amount of antibodies not bound to said bacterium with a carrier or a biosensor as described above.

Preferably the antigen is a serogroup-representing carbohydrate.

This method optionally further comprises the removal of non-bound antibodies from contacted sample and predetermined amount of antibodies by for example washing or immuno-magnetic separation procedures, centrifugation or filtering.

For this type of analysis every type of sample can be used, such as animal feed, manure, feathers, soil, water for consumption or sewage water, meat, orange juice, chocolate, skin, vegetables etc. Animal samples may be obtained from living as well as dead animals.

In this bacteriological assay a single type of antibody as well as a mixture of at least two different types of antibodies (directed against different antigens, for example two different serogroup-representing carbohydrates) is used.

Preferably, such serological and bacteriological assays are performed such that the binding to said carrier or said biosensor is determined by Plasmon Surface Resonance.

The source of the samples is as already outlined above unlimited and may for example be obtained from a human or an animal. Examples of suitable animals are (race) horses, pigs, poultry (for example chicken, turkey, quail, duck, and goose), ruminants (for example calf or cow, goat, sheep). The animals may be farm animals, zoo animals as well as free living animals. Moreover, samples from these animals may be obtained from living as well as dead animals.

In yet another embodiment the invention provides a method for determining the presence of a gram-negative bacterium in a sample comprising
- contacting said sample with target bacteria-specific, bacteriophages and allowing the bacteriophages to infect said sample
- removing non-bound and/or non-invading bacteriophages resulting in a bacteriophage infected sample
- bringing the bacteriophage infected sample into contact with an indicator organism susceptible for the used bacteriophages
- incubate during at least one bacteriophage multiplication cycle
- recover the bacteriophages to obtain a bacteriophage-containing sample
- analyse said bacteriophage-containing sample with a carrier or a biosensor according as described above.

Most analytical methods require prior enrichment and growth in specific media to detect bacteria, including salmonella. Usually sample preparation is very time-consuming relatively to the total analysis time. It generally takes 3 to 5 days before the presence of e.g. salmonella can be confirmed. In many situations, this time for analysis is unacceptable and hinders trade and indirectly threats community health.

The objective of this part of the invention is development of a fast (preferably within 24 h) and/or cost-effective and/or specific and/or sensitive diagnostic method for the determination of the presence of micro organisms. For this reason, the development of a biomolecular interaction assay (BIA) which exploits the ability of genus- and/or serovar-specific bacteriophages to multiply in their 'victim' bacteria, is aimed. An increment in number of the target pathogen-specific phage(s) indicates not only the presence of the target organism but is also a (semi-)quantitative measure for the content of target bacteria in the tested sample.

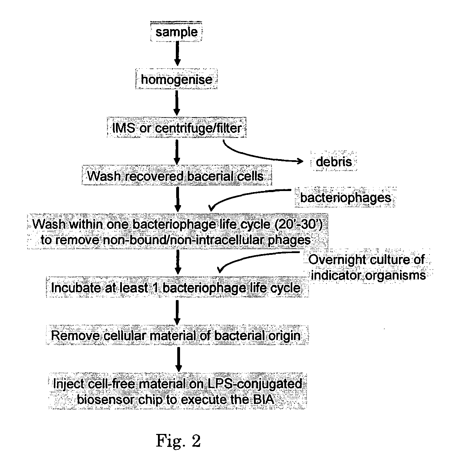

A schematic overview of the proposed BIA method is depicted in Figure 2. A particulate sample is homogenised for example using a Stomacher. Liquid samples are mixed by vigorous shaking. Analyte cells are then extracted or enriched by any suitable method and may comprise (a combination of) selective growth, centrifugation, filtration and/or immuno-magnetic separation (IMS). Enriched cells are fortified with target bacteria-specific bacteriophages and incubated for a few minutes while mixing. Before the multiplication cycle of the bacteriophage is complete, cells are washed to remove as complete as possible any non-bound and non-invading bacteriophages. Following the multiplication cycle of the bacteriophage, the sample is brought in contact with an indicator organism susceptible, i.e. in a life phase that is sensitive for bacteriophage penetration and intracellular multiplication, for the used bacteriophage, preferably at the highest possible concentration (for example concentrated overnight culture). The bacteriophage-bacterium suspension is incubated for at least one bacteriophage multiplication cycle. The phage-infected suspension is then centrifuged or filtered to precipitate/ remove cellular material and to recover multiplied bacteriophages. The bacteriophage-containing sample is injected over an LPS-conjugated biosensor chip (according to the invention) to retain these particles in the detector for the generation of analyte-specific biosensor response.

To gain as much time as possible the indicator organism can be kept as a continuous culture in the lab and has a cell density of usually 10⁹ CFU/ml. Such a suspension may be concentrated to 10¹⁰ CFU/ml, as higher cell densities will increase sensitivity of the proposed method.

This method can be used to determine a single type of serovar but to detect multiple serovars in one run, a mix of different bacteriophages and a mixture of possibly different indicator bacteria may have to be applied.

Target bacteria-specific bacteriophages are described in the prior art and examples are provided in the experimental part, for example anti-*Salmonella enteritidis* bacteriophages.

Phages have been described to attach to LPS, including the phage described in the experimental part for salmonella detection. Suitable carriers/chips are carriers/chips with LPS or with immobilised bacterial surface molecules (thus including membrane proteins and other biomolecules or a combination thereof). Use of LPS of cell membrane material will circumvent the generation of poly- or monoclonal antibodies. If attachment of the phages to bacterial biomolecules (LPS) is not satisfactory in the BIA, biosensor chip-immobilised anti-phage antibodies may have to be used in a successful BIA to capture bacteriophages from the probed sample.

The invention furthermore provides a kit with components suitable for use in any of the described applications. Depending on the customer's demand, such a kit comprises a ready-for use carrier/chip obtained by a method according to the invention. When the customer wants to prepare the carrier himself, the kit will at least comprise (lipo)polysaccharide fortified/enriched with protein (for example haemoglobin or serum albumin) in a predetermined amount, an amount of oxidizing agent (for example periodate), suitable buffers. Optionally, such a kit comprises means for desalting, for example a desalting column. When the customer wants to mix (lipo)polysaccharide and protein himself these components are delivered separately together with an instructions manual. Optionally, such a kit may furthermore comprise positive and/or negative reference sera, a sample dilution buffer and any necessary instruction manual.

The methods as described above are particularly suitable for screening samples on a large-scale basis. In one of the earlier (slow) settings 96 samples were checked within 33 minutes. In a large-scale setting with relative slow biosensor equipment 15.000 samples were screened within 3 months. This number could have been much higher but unfortunately one of the slaughterhouses stopped participating.

The invention will be explained in more detail in the following description, which is not limiting the invention.

### Materials and methods

### 1.1 Materials

### 1.1.1 Chemicals

Amine coupling kits, consisting of *N*-hydroxysuccinimide (NHS), 1-ethyl-3-(3-dimethlylaminopropyl)carbodiimide hydrochloride (EDC) and ethanolamine hydrochloride - sodium hydroxide pH 8.5 and the running buffer (HBS-EP), containing 10 mM HEPES, 150 mM sodium hydrochloride, 3 mM EDTA and 0.005% (v/v) surfactant P20 at pH 7.4, were bought from Biacore AB (Uppsala, Sweden), which also supplied ready-to-use 10 mM glycine and 50 mM sodium hydroxide. Ethanol, ethylene glycol, sodium chloride, sodium hydroxide and trichloroacetic acid (TCA) were purchased from Merck (Darmstadt, Germany). Carboxymethylated-dextran sodium salt, sodium cyanoborohydride and carbohydrazide were obtained from Fluka Chemie GmbH (Buchs, Switzerland). CHAPS (Plus one) was delivered by Pharmacia Biotech (Uppsala, Sweden). Sodium acetate trihydrate and acetic acid were supplied by J.T. Baker (Deventer, The Netherlands). Guanidine hydrochloride was obtained from Calbiochem (San Diego, CA, U.S.A.). *Porcine* haemoglobin (Hb) and myoglobin (Mb), chicken ovalbumin (Ob; 98% grade V), *bovine* serum albumin (BSA; 96% Fraction V), sodium periodate, Tween-20, Tween-80 and Triton X-100 were acquired from Sigma Chemical Company (St. Louis, MO, U.S.A.). Water was obtained from of a Milli Q water purification system (Millipore, Bedford, MA, U.S.A.).

### 1.1.2 Materials

NAP-5 columns (0.5 ml; Sephadex G-25) were purchased from Amersham Biosciences (Roosendaal, The Netherlands) and were used as described by the producer. CM5 biosensor chips were bought from Biacore AB. Dialysis bag (Spectra/Por) with a cut-off of 1 kDa was obtained from Spectrum Laboratories Inc. (Rancho Dominguez, CA, U.S.A.)

### 1.1.3 Anti-salmonella antisera

The following salmonella monovalent 'O' somatic *lapine* antisera were used: anti-O4, anti-05, anti-06,7, anti-08, anti-09, anti-010, anti-012, O Poly E (anti-03, anti-O10, anti-O15, anti-019, anti-034). In addition, salmonella polyvalent 'O' somatic (Poly A-S) *lapine* antisera (anti-02, anti-03, anti-04, anti-05, anti-06,7, anti-08, anti-09, anti-O10, anti-O11, anti-012, anti-O13, anti-O15, anti-016, anti-O17, anti-O18, anti-019, anti-020, anti-021, anti-022, anti-023, anti-028, anti-030, anti-034, anti-035, anti-038, anti-040, anti-041) was used as well. The sera were purchased from Pro-Lab diagnostics (Salmonella Reference Section of the Central Veterinary Laboratory, Weybridge, U.K.). Serogroup specific *murine* anti-B (anti-04, 05 en 027), anti-C (anti-07, 08), anti-D (anti 09, Vi) and anti-E (anti-03, 019) monoclonal antibodies were bought from SIFIN (Berlin, Germany').
Sera were diluted 1:20 (v/v) in HBS-EP containing 1.0 M sodium chloride, 1% (m/v) carboxymethylated dextran and 0.05% (v/v) Tween 80, except anti-05 serum was diluted 1:200 (v/v) and the anti-serogroup specific preparations were diluted 1:100 (v/v) in the same solvent.

### 1.1.4 Reference avian and porcine sera

All reference sera were obtained from the Dutch Animal Health Service (Deventer, The Netherlands). The obtained avian reference sera were reactive with *Salmonella enteritidis* (serogroup D₁), *S. typhimurium* (serogroup B), *S. pullorum*/*gallinarum* (serogroup D₁) and *S. infantis* (serogroup C₁), and were further referred to as C-Se, C-St, C-Spg and C-Si, respectively. These chicken sera were originally prepared for ELISA analyses as positive references. In addition, specific pathogen-free chicken serum (further referred to as C-SPF) was purchased as a negative control reference sample. These sera were reconstituted from freeze-dried material by addition of water at a volume indicated by the manufacturer. C-Se, C-Spg and C-Si were diluted 1:200 (v/v) in HBS-EP containing 1.0 M sodium chloride, 1.0% (m/v) carboxymethylated dextran and 0.05% (v/v) Tween-80, whereas C-SPF and C-St were diluted 1:50 (v/v) in the same solution. Likewise, *porcine* sera from animals challenged with *S. typhimurium* and *S. livingstone* (serogroup C₁) were referenced as P-St and P-S1, respectively. In addition, *Actinobacillus pleuropneumoniae* serotype 2-reacting *porcine* serum used as control in a complement fixation test, was exploited as negative control for porcine serum in the salmonella biosensor assay. The *porcine* sera were diluted 1:20 (v/v) in HBS-EP containing 1.0 M sodium chloride, 1% (m/v) carboxymethylated dextran and 0.05% (v/v) Tween 80 as end concentrations.

### 1.1.5 Salmonella stock

The bacteria *Salmonella goldcoast* (Sg; serogroup C₂), *S. livingstone* (Sl) and *S. melaegridis* (Sm; serogroup E₁) were obtained from an in-house collection, while *S. enteritidis* #23 phage type Pt4 (Se), and *S. typhimurium* X-193 phage type 507 (St) were kind gifts of F. G. van Zijderveld (Animal Sciences Group, Lelystad, The Netherlands). The bacteria were grown in overnight cultures in Nutrient Broth #2 (Oxoid, Basingstroke, U.K.). Stocks of salmonella strains were morphologically and biochemically confirmed as salmonella and also verified for the presence of the correct, expected O-antigens by an agglutination reaction of the cells with specific standard anti O-antigen anti-sera (Pro-Lab diagnostics) as indicated in Table 3 on a glass plate. After addition of a half of the original volume with glycerol (Merck), stocks were stored in portions at -80°C.

**Table 3. O-antigen verification of the salmonella serovars used for LPS production. The expected reaction of anti sera used for verification by agglutination, is given**

| **Salmonella Serovar** | **Agglutination sera** | | | | | | | | |
|---|---|---|---|---|---|---|---|---|---|
| | **α-O4**^{**a**} | **α-O5** | **α-O6,7** | **α-O8** | **α-O9** | **α-O10** | **α-O12** | **polyA-S** | **Poly E** |
| S.*enteritidis* (O9,O12)^{b} | - | - | - | - | + | - | + | + | - |
| S.*goldcoast* (06,8) | - | - | + | + | - | - | - | + | - |
| *S.livingstone* (06,7) | - | - | + | - | - | - | - | + | - |
| *S.meleagridis* (03,010) | - | - | - | - | - | + | - | + | + |
| *S.typhimurium* (04,05,012) | + | + | - | - | - | - | + | + | - |

| | | | | | | | | | |
|---|---|---|---|---|---|---|---|---|---|
| ^{a} α-O4: antibodies reacting with antigen structure coded with 04; in a similar way the antibodies against O5, O6,7, O9, O10 and 012 are indicated. ^{b} O antigens specific for salmonella serovar is indicated in brackets | | | | | | | | | |

### 1.2 Methods

### 1.2.1 Extraction of LPS

Overnight cultures of salmonella were prepared by applying 100 µl from their corresponding stocks on each of the 120 plates containing brain heart infusion agar (BHIa, Oxoid). The presence of the expected salmonella serovar was confirmed through conventional selective growth, bio- and immunochemical classification, whenever new stock suspensions were produced. The bacteria were harvested from the surface of the plates into 1 ml 9 g/l NaCl (saline) solution per agar plate using a trigalski spatula. Each plate was washed twice with 2 ml saline solution. Bacteria were collected in six centrifugation tubes. Each tube was complemented with 100 ml saline and mixed before centrifugation at 10,000 g and 4°C for 15 min and supernatant was discarded. This centrifugation step was repeated twice by suspending cells in 75 ml saline wash solution per tube each run. While kept on ice, pelleted bacteria were suspended in water at a volume ratio, which was a 5-fold to the weight of the bacteria. An equivolume of 0.250 M (Se) or 0.500 M (Sg, Sl, Sm and St) TCA was added to give end concentrations of 0.12 M and 0.25 M, respectively, followed by continuous stirring at 4°C for 3 h. A lipopolysaccharide (LPS)-containing supernatant was then acquired at 20,000 g and 4°C for 30 min. The pH of the supernatant was adjusted to pH 6.5 with 5 M sodium hydroxide and when nearing the aimed pH with 0.10 M sodium hydroxide. The final volume of the LPS-containing solution was determined prior to storage at -18°C for 30 min. The solution was diluted with a double volume of freezing cold absolute ethanol from a -18°C storage place, and incubation was continued overnight at -4°C without stirring in a closed, in house-build device with circulating cold ethylene glycol/water (1:4, v/v). An LPS-containing pellet was obtained after centrifugation at 20,000 g and -4°C for 30 min. The particulate material was suspended in a volume of 0.5 ml water per gram original bacterial mass weighed at the start of extraction process. The suspension was dialyzed in a 1-kDa dialysis bag against water at 4°C for two days with regular intermittent refreshment of the water. The bag content was centrifuged at 20,000 g and at 4°C for 30 min, and the supernatant was lyophilized. The lyophilisate was weighed to establish the recovery of LPS. LPS was reconstituted in water to make up an end concentration of 5 mg/ml. Dependent of type of LPS and batch (see also section 1.2.2), a volume of 1 mg/ml *porcine* haemoglobin (Hb) was added to a concentration as indicated in the text. Each batch was portioned into 0.5-mg LPS fractions, which were dried using a vacuum evaporator and then stored at 5-8°C.

### 1.2.2 Optimal haemoglobin content

Protein was added to an LPS preparation prior to its chemical modification and immobilization to a sensor chip to acquire high coating levels and high serum responsive antigens. The optimum Hb content in each LPS batch was established by comparison of the responses of immobilized LPS that was fortified with Hb at different levels, using a panel of positive and negative reference sera.

### 1.2.3 Oxidation of LPS

A portion of 0.5 mg haemoglobin-fortified LPS was dissolved in 500 µl 100 mM sodium acetate pH 5.5. Following the addition of 20 µl 50 mM sodium periodate, the solution was incubated for 40 min on ice protected from light. The oxidation of LPS was quenched and the solution was desalted by passing 500 µl of the reaction mixture through an NAP-5 cartridge with a gravity-controlled flow. Modified LPS was eluted with 1 ml 10 mM sodium acetate, pH 4.0. Prior to use, the cartridge was conditioned thrice with 3 ml 10 mM sodium acetate, pH 4.0.

### 1.2.4 Immobilization of LPS

To immobilize the antigens to a sensor chip, the following handlings were conducted at a flow rate of 5 µl/min in a Biacore 3000 instrument controlled by Biacore 3000 Control Software (version 3.1.1; Biacore). Immobilization of oxidized LPS was achieved by execution of the aldehyde-coupling procedure described in BIAapplications Handbook, version AB (1998). Briefly, the dextran layer at the biosensor chip CM5 was activated with a 7-min pulse of a mixture of EDC/NHS available from the amine-coupling kit. The activation was immediately followed by injection of 5 mM aqueous carbohydrazide for 7 min as well.
Deactivation of the excess of reactive groups was then accomplished with a pulse of 1 M ethanolamine for 7 min. Prior to immobilisation of the antigen, LPS was diluted in sodium acetate pH 4.0 in a ratio dependent of the salmonella serovar (see text) and immobilised for 32 min. The linkage between dextran-matrix and antigen was then stabilized by injection of 100 mM sodium cyanoborohydride solved in 10 mM sodium acetate at pH 4 at a flow rate of 2 µl/min for 20 min. A relative response indicative for a successful LPS immobilisation procedure is 2 kRU for a 62.5 µg/ml LPS solution containing 15% (m/m) protein, and 9 kRU for a 250 µg/ml LPS solution containing 50% (m/m) protein.

### 1.2.5 SPR biosensor assay

Optical SPR biosensor assays were performed on a Biacore 3000 SPR biosensor platform controlled by the same software as described above. Prior to injection, sera were diluted in HBS-EP buffer containing 1.0% (m/v) carboxymethylated-dextran sodium salt, 1.0 M sodium chloride and 0.05% (m/v) Tween 80 at a ratio of 1:50 (v/v) or otherwise as indicated in the text. The mixtures were incubated for at least 2 min at ambient temperature. Pig sera were injected for 2 min at 40 µl/min, whereas bird sera were injected for 2 min at 5 µl/min or 20 µl/min as indicated.

Regeneration of the chip to recover the antigenic activity of the sensor surface was achieved with a 15-s pulse of 6 mM glycine at pH 2, containing 6 M guanidine hydrochloride, 0.1% (m/v) CHAPS, and 0.1% (v/v) of each Tween-20, Tween-80 and Triton X-100. This was followed with a second regeneration step with the running HBS-EP buffer enriched with 0.05% (m/v) CHAPS (end concentration) for 12 s at 100 µl/min.

### 1.2.6 Monosaccharide analysis

Trimethylsilylated (methyl ester) methyl glycosides were prepared from the glycan samples by methanolysis (1.0 M methanolic HCl, 24 h, 85°C) followed by re-N-acetylation and trimethylsilylation, and then analyzed by gas chromatography/mass spectrometry as described [Kamerling JP, Vliegenthart JFG (1989)]. The quantitative analysis was carried out by gas chromatography on a capillary EC-1 column (30 m x 0.32 mm, Alltech) using a Chrompack CP 9002 gas chromatograph operated with a temperature program from 140°C to 240°C at 4°C/min, and flame-ionization detection. The identification of the monosaccharide derivatives was confirmed by gas chromatography/mass spectrometry on a Fisons Instruments GC 8060/MD 800 system (Interscience) equipped with an AT-1 column (30 m x 0.25 mm, Alltech).

### Results

### LPS isolation

For the production of LPS, yields of bacterial cells and of LPS were compared for agar plate culture and growth of salmonella in broth (Table 4). For laboratory technical reasons, it was decided to harvest bacteria from agar plates, rather than isolation of the cells from culture flasks. The results of the isolation of LPS from Se, Sg, Sl, Sm and St are summarized in Tables 5 to 9, respectively. The standardized isolation of well-defined LPS is determinative for a successful and robust serological assay. To secure assay performance, batch-to-batch differences should be kept to a minimum. For this reason, several batches of LPS extracted from each Se, Sg, Sl, Sm and St were produced. The recovery of LPS largely depended on the final TCA concentration in the mixture during extraction of LPS (cf. Table 6 and Table 8), although this relationship was not completely clear for the extraction of LPS from St (Table 9). Indeed, no accurate optimal TCA concentration could be determined for each LPS type through the testing of a broad range of TCA concentrations. Here, optimal TCA would yield highest LPS amounts, and give highest specific serological and lowest aspecific biosensor responses. In this study, the TCA concentration chosen as 'optimal' for LPS extraction from the different salmonella serotypes was based on the final LPS yields after dialysis, and were 0.12 M, 0.25 M, 0.25 M, 0.25 M and 0.25 M as end concentrations for Se, Sg, Sl, Sm and St, respectively.

**Table 4. Recovery of LPS from S. enteritidis cells grown either as a suspension in a bioreactor containing so-called nutrient broth#2 (broth) or on BHI agar plates (agar). LPS was isolated using indicated TCA end concentrations. The yield of LPS relative to the amount of isolated cells is indicated in the last column.**

| **LPS Batch code** | **Culture method** | **TCA (M)** | **bacteria yield (g)** | **recovered LPS (mg)** | **LPS yield (%, m/m)** |
|---|---|---|---|---|---|
| Se01 | Broth | 0.25 | 3,9 | 16 | 0,42 |
| Se02 | Broth | 0.25 | 5,0 | 21 | 0,41 |
| Se03* | Agar | 0.25 | 14 | 0,2 | 0,00 |
| Se04a | Broth | 0.25 | 3,4 | 0,4 | 0,01 |
| Se04b | broth | 0.5 | 3,4 | 2 | 0,06 |
| Se05** | agar | 0.5 | 7,6 | 2,4 | 0,03 |
| Se06a | agar | 0.5 | 8,8 | 3,9 | 0,04 |
| Se06b | agar | 0.25 | 7,6 | 9,5 | 0,12 |
| Se06c | agar | 0.125 | 8,9 | 13 | 0,14 |
| Se07a | agar | 0.1 | 9,1 | 12 | 0,13 |
| Se07b | agar | 0.05 | 9,2 | 2,9 | 0,03 |
| Se07c | agar | 0.025 | 9,3 | 4 | 0,04 |
| Se2003.1 | agar | 0.125 | 29 | 48 | 0,16 |
| Se2003.2 | agar | 0.125 | 17 | 25 | 0,15 |
| Se2003.4 | agar | 0.125 | 13 | 5,1 | 0,04 |
| Se2005.1 | agar | 0.25 | 15 | 18 | 0,13 |

| | | | | | |
|---|---|---|---|---|---|
| * pH of TCA-containing mixture is outlying ** some material was lost during sample work up process. | | | | | |

**Table 5. Recovery of Salmonella enteritidis cells grown on BHIa plates. LPS was isolated using 0.12 M TCA end concentration (cf. Table 4). Rec, recovered.**

| **LPS Batch (Se)** | **Total bacterial yield (g)** | **Number of BHIa plates** | **bacteria per plate (g)** | **Rec. LPS/cells (% m/m)** |
|---|---|---|---|---|
| Se2003.1 | 29.09 | 98 | 0.29 | 0.16 |
| Se2003.2 | 17.27 | 60 | 0.28 | 0.15 |
| Se2003.4 | 13.00 | 40 | 0.32 | 0.04 |
| Se2005.1 | 44.5 | 120 | 0.37 | 0.12 |

**Table 6. Recovery of Salmonella goldcoast cells grown on BHIa plates. LPS was extracted using a TCA end concentration as indicated. Rec, recovered.**

| **LPS Batch code** | **Total bacterial yield (g)** | **Number of BHIa plates** | **bacteria per plate (g)** | **TCA**^{**a**} **(M)** | **Rec. LPS/cells (% m/m)** |
|---|---|---|---|---|---|
| Sg2003.1 | 40.39 | 120 | 0.34 | 0.075 | 0.01 |
| Sg2003.2 | 35.09 | 120 | 0.29 | 0.25 | 0.41 |
| Sg2003.3 | 12.89 | 40 | 0.32 | 0.25 | 0.36 |
| Sg2005.1 | 46.99 | 120 | 0.39 | 0.25 | 0.30^{b} |

| | | | | | |
|---|---|---|---|---|---|
| ^{a} end concentration TCA in extraction mixture. ^{b} approximately a third of the production was lost during work-up. | | | | | |

**Table 7. Recovery of Salmonella livingstone cells grown on BHIa plates. LPS was extracted using 0.250 M TCA end concentration. Rec, recovered.**

| **LPS Batch code** | **Total bacterial yield (g)** | **Number of BHIa plates** | **bacteria per plate (g)** | **Rec. LPS/cells (% m/m)** |
|---|---|---|---|---|
| S12003.1 | 32.89 | 120 | 0.27 | 0.52 |
| S12003.2 | 13.63 | 40 | 0.34 | 0.51 |
| S12005.1 | 47.40 | 120 | 0.40 | 0.64 |

**Table 8. Recovery of Salmonella meleagridis cells grown on BHIa plates. LPS was isolated using a TCA end concentration as indicated. Rec, recovered.**

| **LPS Batch code** | **Total bacterial yield (g)** | **Numberof BHIa plates** | **Bacteria per plate (g)** | **TCA**^{**a**} **(M)** | **Rec. LPS/cells (% m/m)** |
|---|---|---|---|---|---|
| Sm2003.1a^{b} | 9.10 | 30 | 0.30 | 0.250 | 0.32 |
| Sm2003.1b^{b} | 10.13 | 30 | 0.34 | 0.125 | 0.19 |
| Sm2003.1c^{b} | 10.00 | 30 | 0.33 | 0.075 | 0.06 |
| Sm2003.2^{b} | 40.42 | 120 | 0.33 | 0.075 | 0.02 |
| Sm2003.3 | 37.28 | 138 | 0.27 | 0.250 | 0.47 |

| | | | | | |
|---|---|---|---|---|---|
| ^{a} end concentration TCA in extraction mixture. ^{b} batches Sm2003.1 to 2003.2 were combined to a single batch called Sm2003.1 | | | | | |

**Table 9. Recovery of Salmonella typhimurium cells grown on BHIa plates. LPS was isolated using 0.250 M TCA end concentration. Rec, recovered.**

| **Batch code** | **Total bacterial yield (g)** | **Number of BHIa plates**^{**a**} | **bacteria per plate (g)** | **TCA**^{**a**} **(M)** | **Rec. LPS/cells (% m/m)** |
|---|---|---|---|---|---|
| St2003.1 | 28.51 | 69 | 0.41 | 0.125 | 0.18 |
| St2003.2^{b} | 39.21 | 120 | 0.33 | 0.250 | 0.06 |
| St2003.3^{b} | 16.6 | 56 | 0.30 | 0.125 | 0.09 |
| St2003.4a^{b} | 18.15 | 60 | 0.30 | 0.250 | 0.18 |
| St2003.4b^{b} | 19.2 | 60 | 0.32 | 0.125 | 0.06 |
| St2005.1 | 46.80 | 120 | 0.39 | 0.250 | 0.19 |

| | | | | | |
|---|---|---|---|---|---|
| ^{a} end concentration TCA in extraction mixture; ^{b} batches St2003.2 to St2003.4b were combined to a single batch called St2003.2 | | | | | |

The monosaccharide composition of isolated LPS preparations were analyzed to reveal the consistency of the isolation and purification procedure for LPS from different salmonella growths. It must be noted that analyses were performed on LPS preparations that were ready for oxidation and for that reason fortified with Hb at levels that were determined most optimal for the LPS batch tested (see below). For this purpose, GC-FID and GC-MS analyses were carried out after methanolysis of the Hb-fortified LPS preparations (Table 10 through Table 14). These results show that Hb does not contribute to a significant amount of carbohydrates in the final LPS preparation. Analysis of BHIa, showed the presence of exclusively galactose (Gal) and glucose (Glc). The content of these monosaccharides was 5.6 µg/mg dried BHIa. Analyses of the salmonella LPS preparations, demonstrated the occurrence of Gal, Glc, *N*-acetyl glucosamine (GlcNAc), glycero-manno-heptose (Hep), 2-keto-3-deoxy-octonic acid (KDO), mannose (Man) and rhamnose (Rha; 6-deoxy-mannose) in accordance with their carbohydrate structures. Their relative occurrence was expressed as a molar ratio relative to 1.0 Man (as part of the PS region) or relative to 3.0 Hep (as part of the core region). It should, however, noted that the core region contains 2 or 3 Hep residues.
Furthermore, GlcNAc can originate from either GlcNAc as in the repeating unit of Sl LPS, or from glucosamine (GlcN), which occurs as disaccharide in the lipid A moiety as backbone for the attached lipids. Gal occurs in the core region, which is conserved in all *Salmonella enterica* serovars, and in the PS region of Se, Sg, St and Sm as well. In these cases, the molar ratio of Gal is expected to be in excess of 1.0 Man, except for Sg in which each repeating unit contains 2 Man residues. The monosaccharide analyses did not include the detection of O-acetylated, posphoryl-ethanolaminated or phosphorylated constituents, nor that of abequose (Abe; 3,6-dideoxy-xylohexose) or tyvelose (Tyv; 3,6-dideoxy-arabinose), which occur in the polysaccharide and core regions of the isolated LPS types as well.
Analysis of Se LPS, showed the occurrence of Gal, Man and Rha at a molar ratio of 1.4, 1.0 and 1.2, respectively, in batch Se2003.1, whereas this ratio was 1.1, 1.0 and 0.9, respectively, in batch Se2003.4 (Table 5). This ratio is in good compliance with the composition of a repeating unit as [Tyv-]Man-Rha-Gal, except the Rha ratio was significantly too high in batch Se2003.1. The carbohydrate content calculated on the basis of determined monosaccharides, was significant higher in batch Se2003.4, namely 241 µg compared 123 µg of batch Se2003.1. Considering the occurrence of 2 GlcN residues in the lipid A and a single GlcNAc residue in the core region and a single Man residue in each repeating unit, the number of repeating units was estimated 19 and 20 in batches Se2003.1 and Se2003.4, respectively.

Monosaccharide analysis of oxidized Se2003.1 clearly demonstrates significant differences with the non-oxidized identical batch (Table 10). In contrast to the two GlcN residues, it is expected that the non-reducing, terminal GlcNAc residue is for the greater part oxidized. Alditol derivatives were not detected by the monosaccharide analysis applied and a corresponding amount of GlcNAc-ol was not determined. As Gal and Man in the repeating unit are not susceptible towards periodate oxidation, the molar ratio of Man in the oxidized batch is normalized to that of Man in the non-oxidized batch. It should be noted that both Gal residues in the core region are susceptible towards oxidation and thus the total Gal ratio is affected. Inspection of the molecular structure of Se LPS, suggests that in addition to terminal GlcNAc and core Gal, terminal KDOII-KDOIII disaccharide, and conjugated HepI and terminal HepIII are susceptible to periodate oxidation as well. Indeed, the molar ratios of these monosaccharide residues suggest the loss of one Hep residue and approximately 1.6 KDO residues. It should be noted that KDOII may not be completely oxidized when this residue is conjugated with a posphoryl-ethanolamine group.

**Table 10. Monosaccharide analysis of S. enteritidis LPS and of oxidized S. enteritidis LPS. LPS was fortified with Hb at 15% (m/m). Molar ratios were determined on the basis of two GlcN and one GlcNAc residues (detected as three GlcNAc residues) present in the core and lipid A regions (referred to as CORE) and on the basis one Man residues in the repeating unit (referred to as UNIT). Normalized GlcNAc and Man residues are indicated by underlining. Carbohydrate content was determined in 0.5 mg LPS preparations, except monosaccharide analysis was performed on 125 mg oxidized material.**

| **Monosaccharide** | **Molar ratio** | | | | | |
|---|---|---|---|---|---|---|
| | **Batch Se2003.1** | | **Batch Se2003.1 (oxidized)** | | **Batch Se2003.4** | |
| | CORE | UNIT | CORE | UNIT | CORE | UNIT |
| Gal | 29.7 | 1.4 | 28.7 | 1.3 | 26.3 | 1.1 |
| Glc | 6.3 | 0.3 | 7.5 | 0.4 | 6.9 | 0.3 |
| GlcNAc | 3.0 | + 2.2 | | + 3.0 | | + |
| Hep | 2.8 | + | 1.9 | + | 2.0 | + |
| KDO | 3.2 | + | 1.4 | + | 2.3 | + |
| Man | 21.6 | 1.0 | 21.6 | 1.0 | 23.5 | 1.0 |
| Rha | 24.8 | 1.2 | 24.4 | 1.1 | 20.9 | 0.9 |
| Carbohydrate content (µg)^{a} | 123.0 | | --^{b} | | 241 | |
| Nr of repeating units | 19 | | - - | | 20 | |

| | | | | | | |
|---|---|---|---|---|---|---|
| ^{a} does not include the contribution of Tyv residues; ^{b} Amount of LPS-containing material analysed was not accurately determined. | | | | | | |

Compared to Se LPS, monosaccharide analysis of Sg LPS suggests that LPS structure were smaller as the number of repeating units was significant lower (Table 11). The relative contribution of core Gal to PS Gal is for that reason larger and total molar ratio is found 1.5. In a similar way, the molar ratio for Glc is found at 1.3 (batch Sg2003.2) and 1.5 (batch Sg2003.3), whereas the molar ratio for Rha fits with the expected structure. Batch Sg2003.2 seems however to contain less terminal HepIII and terminal KDOIII and could therefore offer less possibility for immobilization to the sensor chip.

**Table 11. Monosaccharide analysis of LPS isolated from S. goldcoast fortified with Hb at 50% (m/m). Molar ratios were determined on the basis of two GlcN and one GlcNAc residues (detected as three GlcNAc residues) present in the core and lipid A regions (referred to as CORE) and on the basis two Man residues in the repeating unit (referred to as UNIT). Normalized GlcNAc and Man residues are indicated by underlining. Carbohydrate content was determined in 0.5 mg LPS preparations.**

| **Monosaccharide** | **Molar ratio** | | | |
|---|---|---|---|---|
| | **Batch Sg2003.2** | | **Batch Sg2003.3** | |
| | CORE | UNIT | CORE | UNIT |
| Gal | 15.3 | 1.5 | 14.8 | 1.5 |
| Glc | 13.5 | 1.3 | 14.3 | 1.5 |
| GlcNAc | 3.0 | + | 3.0 | + |
| Hep | 2.9 | + | 2.3 | + |
| KDO | 3.0 | + | 2.8 | + |
| Man | 20.5 | 2.0 | 19.2 | 2.0 |
| Rha | 10.6 | 1.0 | 10.2 | 1.0 |
| Carbohydrate content (µg)^{a} | 170 | | 199 | |
| Nr of repeating units | 9 | | 8 | |

| | | | | |
|---|---|---|---|---|
| ^{a} does not include the contribution of Abe residues. | | | | |

Normalisation of the number of core residues from the monosaccharide analysis results of Sl LPS (Table 12) was hampered by the occurrence of GlcNAc in the repeating units of the PS. When the number of Hep residues was set at 3.0, an unacceptable overestimation of the number of KDO residues arose. For that reason, the number of Hep was set at 2.0, but may need to be modified, so that the number of KDO is closer to 3.0. As the number of Man residues in each repeating unit is four, molar ratios were corrected for 4.0 Man residues. On the basis of a molar ratio of 4:1 of Man/GlcNAc in the PS region, the number of repeating units was calculated on the basis of the remaining core GlcNAc and Lipid A GlcN residues. This calculation revealed that the number of repeating units in Sl was also relatively small, namely 8 and 10 units in batch S12003.1 and batch2003.2, respectively.

**Table 12. Monosaccharide analysis of LPS isolated from S. livingstone fortified with Hb at 50% (m/m). Molar ratios were determined on the basis of two Hep residues present in the core (referred to as CORE) and on the basis four Man residues in the repeating unit (referred to as UNIT). Normalized GlcNAc and Man residues are indicated by underlining. Carbohydrate content was determined in 0.5 mg LPS preparations. n.d., not detected.**

| **Monosaccharide** | **Molar ratio** | | | |
|---|---|---|---|---|
| | **Batch S12003.1** | | **Batch S12003.2** | |
| | CORE | UNIT | CORE | UNIT |
| Gal | 3.5 | 0.4 | 5.1 | 0.4 |
| Glc | 17.4 | 1.7 | 23.5 | 1.6 |
| GlcNAc | 14.0 | 1.4 | 18.7 | 1.3 |
| Hep | 2.0 | 0.2 | 2.0 | 0.14 |
| KDO | 2.7 | 0.3 | 2.7 | 0.2 |
| Man | 40 | 4.0 | 57 | 4.0 |
| Rha | n.d. | n.d. | n.d. | n.d. |
| Carbohydrate content (µg) | 212 | | 239 | |
| Nr of repeating units | 8 | | 10 | |

**Table 13. Monosaccharide analysis of S. meleagridis LPS. Batches Sm2003.1 and Sm2003.3 were fortified with 50% (m/m) Hb. Molar ratios were determined on the basis of two GlcN and one GlcNAc residues (detected as three GlcNAc residues) present in the core and lipid A regions (referred to as CORE) and on the basis one Man residues in the repeating unit (referred to as UNIT). Normalized GlcNAc and Man residues are indicated by underlining. Carbohydrate content was determined in 0.5 mg LPS preparations.**

| **Monosaccharide** | **Molar ratio** | | | |
|---|---|---|---|---|
| | **Batch Sm2003.1** | | **Batch Sm2003.3** | |
| | CORE | UNIT | CORE | UNIT |
| Gal | 20.4 | 1.5 | 22.4 | 1.4 |
| Glc | 7.9 | 0.6 | 4.8 | 0.3 |
| GlcNAc | 3.0 | + | 3.0 | + |
| Hep | 2.8 | + | 2.9 | + |
| KDO | 2.8 | + | 2.9 | + |
| Man | 14.2 | 1.0 | 15.4 | 1.0 |
| Rha | 16.1 | 1.1 | 17.6 | 1.2 |
| Carbohydrate content (µg)^{a} | 170 | | 220 | |
| Nr of repeating units | 12 | | 13 | |

| | | | | |
|---|---|---|---|---|
| ^{a} does not include the contribution of *O*-acetyl groups, which may be attached to the repeating Gal residues. | | | | |

Monosaccharide analysis of Sm LPS (Table 13) showed a completely different composition as that for Sl LPS in accordance with its molecular structure containing Man-Rha-Gal repeating units. As described above, the molar ratio for Gal is more than the expected 1.0 in the repeating unit partly by the contribution of Gal residues in the core region.

Likewise, equimolar ratios are expected for Glc, Man, Rha and Gal as these residues form a repeating unit in St LPS (Table 14). It should be noted here that abequose is also part of the repeating unit, but is not in the analysis applied. Batch St2003.2, however, contains less oxidizable Hep and KDO, which may affect the efficacy of the immobilization of LPS from this preparation. At the other hand, Batch St2003.2 contains much more carbohydrate than batch St2003.1, namely 249 µg relative to 154 µg in 0.5 mg LPS, respectively.

**Table 14. Monosaccharide analysis of S. typhimurium LPS. Batches St2003.1 and St2003.2 were fortified with Hb at 15% (m/m) and 25% (m/m), respectively. Molar ratios were determined on the basis of two GlcN and one GlcNAc residues (detected as three GlcNAc residues) present in the core and lipid A regions (referred to as CORE) and on the basis one Man residues in the repeating unit (referred to as UNIT). Normalized GlcNAc and Man residues are indicated by underlining. Carbohydrate content was determined in 0.5 mg LPS preparations.**

| **Monosaccharide** | **Molar ratio** | | | |
|---|---|---|---|---|
| | **Batch St2003.1** | | **Batch St2003.2** | |
| | CORE | UNIT | CORE | UNIT |
| Gal | 24.7 | 1.4 | 24.2 | 1.4 |
| Glc | 17.0 | 1.0 | 16.2 | 1.0 |
| GlcNAc | 3.0 | + | 3.0 | + |
| Hep | 2.8 | + | 2.6 | + |
| KDO | 2.7 | + | 2.4 | + |
| Man | 20.5 | 1.0 | 19.2 | 1.0 |
| Rha | 19.9 | 1.1 | 19.5 | 1.2 |
| Carbohydrate content (µg)^{a} | 154 | | 249 | |
| Nr of repeating units | 15 | | 14 | |

| | | | | |
|---|---|---|---|---|
| ^{a} does not include the contribution of (*O*-acetylated) Abe residues. | | | | |

### Protein-supported immobilization of LPS

Unexpectedly, intact LPS poorly coupled through its KDO-carboxylic acid function to EDC/NHS-activated carboxymethated dextran, and no significant responses of reference sera were observed. To improve immobilization of LPS to the sensor chip, LPS was oxidized using sodium periodate to create reactive aldehyde groups in its carbohydrate constituents, which would allow the so-called aldehyde coupling procedure, *i*.*e*. condensation of aldehyde with a hydrazide function into a hydrazone linkage followed by reduction to a hydrazide product. Without oxidation, LPS had indeed little potential to immobilize to a surface of a CM5 chip coated with carbohydrazide (results not shown).

Coupling of oxidized LPS, however, gave disappointing reactivity with reference sera, probably as a result of insufficient immobilization of the antigens. Commercially acquired phenol-extracted LPS, either intact or detoxified (*i*.*e*. cleavage of lipid A), from *S*. *enteritidis* gave low responses when immobilized after oxidation, namely 187 RU and 167 RU, respectively. It must be noted, however, that besides poor coupling, oxidation may have destroyed a part of the antigenic structures, which may give poor serological responses. The degree of oxidation was investigated by monosaccharide analysis of Se LPS (Table 10). This analysis revealed that relative amounts of KDO, Hep and GlcNAc, which are constituent of the core region and not of the repeating antigenic units in the PS part, were significantly reduced compared to non-oxidized Se LPS. Importantly, monosaccharide residues part of the PS, and thus antigenic structures, apparently remained intact under the mild oxidation conditions, which were applied.

**Table 15. Biosensor responses following immobilization and responses of reference antisera flowed over chip surfaces, which were prepared with oxidized and non-oxidized Se or St LPS in the presence of 15% (m/m) porcine Hb.**

| **Type of LPS** | **Periodate treatment** | **Biosensor response (RU)** | | | | | |
|---|---|---|---|---|---|---|---|
| | | **Level of immobilization** | **α-O4**^{**a**} | **α-O5** | **α-O9** | **α-O12** | **O poly A-S** |
| Se | No | 6515 | 1 | 9 | 6 | 9 | 4 |
| St | No | 4402 | 2 | 156 | 1 | 5 | 0 |
| Se | yes | 4265 | -21 | -3 | 77 | 202 | 159 |
| St | yes | 5950 | 302 | 5005 | -12 | 225 | 137 |

| | | | | | | | |
|---|---|---|---|---|---|---|---|
| ^{a} anti-serum against indicated O antigen was tested | | | | | | | |

**Table 16. Biosensor responses following immobilization and responses of reference antisera flowed over chip surfaces, which were prepared with oxidized St LPS (batch St2003.1) in the presence of 7% (m/m) of the indicated protein.**

| **Protein added** | **Biosensor response (RU)** | | | | | | | | | | | |
|---|---|---|---|---|---|---|---|---|---|---|---|---|
| | **Level of immob.**^{**a**} | **O4** | **O5** | **O9** | **O12** | **O poly E** | **O poly A-S** | **C-SPF** | **C-St** | **C-Se** | **C-Si** | **C-Spg** |
| BSA^{b} | 344 | n.d^{c} | n.d | n.d | n.d | n.d | n.d | n.d | n.d. | n.d. | n.d. | n.d. |
| BSA | 7450 | 19 | 216 | 1 | 18 | 4 | 14 | 5 | 18 | 21 | 12 | 42 |
| Hb | 3410 | 319 | 3874 | 4 | 192 | 3 | 203 | 11 | 151 | 140 | 51 | 762 |
| Mb | 815 | 59 | 773 | 3 | 45 | 2 | 47 | 8 | 46 | 34 | 20 | 152 |
| Ob | 5540 | 76 | 926 | 10 | 56 | 12 | 56 | 12 | 47 | 45 | 25 | 177 |

| | | | | | | | | | | | | |
|---|---|---|---|---|---|---|---|---|---|---|---|---|
| ^{a} level of immobilization; ^{b} BSA was added to oxidized and desalted LPS; ^{c} immobilization of LPS was considered too low for further reference sera analysis. | | | | | | | | | | | | |

To optimize the binding of LPS and consequently improve detection of binding antibodies from sera, oxidation of LPS was then executed in the presence of a protein to allow the formation of protein-LPS complexes through Schiff-base reactions between proteinaceous amines and aldehyde functions of LPS. Indeed, commercially available TCA extracted Se LPS, containing considerable amounts of bacterial proteins, gave improved immobilization at 562 RU compared to phenol-extracted and ion-exchange chromatography-purified Se LPS at 208 RU.
Oxidation of LPS was necessary, as mixtures containing non-oxidized LPS and protein show relatively high immobilization levels but insignificant specific responses (Table 15). In addition, protein addition was only beneficial prior to oxidation of LPS, as addition of BSA to oxidized and desalted St LPS gave acceptable immobilization levels but no expected serological responses (Table 16). In a similar way, Hb yielded relatively high immobilization levels but no serological responses, as expected (results not shown).

For method improvement purposes, proteins with relatively high degree of homology of their primary and secondary structure between homeothermic vertebrate species and occurring in serology-suitable matrices were selected for further investigations. For that reason, the performance of chicken ovalbumin, *porcine* haemoglobin, *bovine* serum albumin or *porcine* myoglobin fortified (7%, m/m) St LPS was compared (Table 16). Haemoglobin gave clearly best improvement of immobilization levels together with best expected antigen-antibody reactivity profile. In the presence of Hb, in particular, 012, poly O A-S and C-St reference sera gave better responses.

This experiment was repeated with the addition of BSA, Hb and Mb at levels indicated in Tables 17 to 20 using batches Se2005.1, Sg2005.1, S12005.1 and St2005.1. This time, 04 and 05 bound to immobilized St LPS as expected (Table 20). Evaluation of these results summarized in Tables 16 to 20 revealed that when considering all expected responses simultaneously per LPS type, the addition of Hb gave highest specific responses compared to the addition of BSA and Mb. Furthermore, in most cases standard deviations that occurred with Hb as supportive protein, were more favorable than those for the addition of BSA and Mb. Haemoglobin was, therefore, selected for further experimentation.
It was observed that the O poly A-S anti sera probably contains a low anti-serogroup C₁ and C₂ titers, as in the case of testing immobilized Sg LPS (Table 18) and Sl LPS (Table 19) relatively low responses are found.

**Table 17. Biosensor responses in response units (RU) following immobilization and responses of reference antisera flowed over chip surfaces, which were prepared with Se LPS oxidized in the presence of 15% (m/m) of the indicated protein. Values were corrected for the C-SPF responses, which are listed as well. Standard deviations are indicated in brackets (N = 5, except for chicken and swine sera N = 4).**

| protein | Immob. level^{a} | Antiserum tested | | | | | | | | |
|---|---|---|---|---|---|---|---|---|---|---|
| | | O9 | O12 | O poly A-S | Anti-serogroup D | C-Se | C-St | C-Spg | P-St | C-SPF |
| Hb | 3403 | 72 (4) | 268 (1) | 160 (2) | 187 (3) | 197 (6) | 55 (7) | 1753 (5) | 76 (13) | 87 |
| Mb | 4228 | 47 (4) | 242 (3) | 133 (5) | 158 (4) | 167 (9) | 40 (12) | 1674 (6) | 80 (16) | 107 |
| BSA | 4883 | 31 (11) | 185 (2) | 99 (16) | 126 (5) | 126 (11) | 28 (30) | 1277 (4) | 68 (24) | 74 |

| | | | | | | | | | | |
|---|---|---|---|---|---|---|---|---|---|---|
| ^{a} level of immobilization. | | | | | | | | | | |

**Table 18. Biosensor responses in response units (RU) following immobilization and responses of reference antisera flowed over chip surfaces, which were prepared with Sg LPS oxidized in the presence of 50% (m/m) of the indicated protein. Values were corrected for the C-SPF responses, which are listed as well. Standard deviations are indicated in brackets (N = 5, except for the chicken and swine sera N = 4).**

| protein | Immob. level^{a} | Antiserum tested | | | | | |
|---|---|---|---|---|---|---|---|
| | | O6,7 | O8 | O poly A-S | Anti-serogroup C | P-S1 | C-SPF |
| Hb | 5542 | 318 (1) | 249 (11) | 69 (3) | 145 (10) | 52 (17) | -1 |
| Mb | 9880 | 237 (1) | 211 (12) | 45 (7) | 81 (26) | 162 (34) | 0 |
| BSA | 10344 | 196 (4) | 124 (8) | 19 (24) | 61 (15) | 110 (22) | -6 |

| | | | | | | | |
|---|---|---|---|---|---|---|---|
| ^{a} level of immobilization. | | | | | | | |

**Table 19. Biosensor responses in response units (RU) following immobilization and responses of reference antisera flowed over chip surfaces, which were prepared with Sl LPS oxidized in the presence of 50% (m/m) of the indicated protein. Values were corrected for the C-SPF responses, which are listed as well. Standard deviations are indicated in brackets (N = 5, except for chicken and swine sera N = 4).**

| protein | Immob. level^{a} | Antiserum tested | | | | | |
|---|---|---|---|---|---|---|---|
| | | O6,7 | O poly A-S | Anti-serogroup C | C-Si | P-S1 | C-SPF |
| Hb | 8180 | 125 (21) | 16 | 167 (4) | 377 (12) | 46 (7) | -17 |
| Mb | 10819 | 97 (24) | 8 | 115 (0) | 349 (3) | 96 (26) | -24 |
| BSA | 11364 | 24 (5) | -33 | 40 (5) | 200 (2) | 68 (20) | 76 |

| | | | | | | | |
|---|---|---|---|---|---|---|---|
| ^{a} level of immobilization | | | | | | | |

**Table 20. Biosensor responses in response units (RU) following immobilization and responses of reference antisera flowed over chip surfaces, which were prepared with St LPS oxidized in the presence of 15% (m/m) of the indicated protein. Values were corrected for the C-SPF responses, which are listed as well. Standard deviations are indicated in brackets (N = 5, except for chicken and swine sera N = 4).**

| protein | Immob. level^{a} | Antiserum tested | | | | | | | | |
|---|---|---|---|---|---|---|---|---|---|---|
| | | O4 | O5 | O12 | O poly A-S | Anti-serogroup B | C-St | C-Spg | P-St | C-SPF |
| Hb | 3355 | 419 (3) | 494 (7) | 212 (2) | 107 (1) | 809 (5) | 398 (14) | 619 (24) | 272 (5) | 9 |
| Mb | 2826 | 353 (8) | 432 (4) | 176 (8) | 80 (9) | 722 (2) | 356 (20) | 532 (24) | 246 (14) | 17 |
| BSA | 5588 | 388 (7) | 304 (7) | 158 (16) | 82 (16) | 542 (4) | 324 (24) | 324 (16) | 244 (17) | 4 |

| | | | | | | | | | | |
|---|---|---|---|---|---|---|---|---|---|---|
| ^{a} level of immobilization. | | | | | | | | | | |

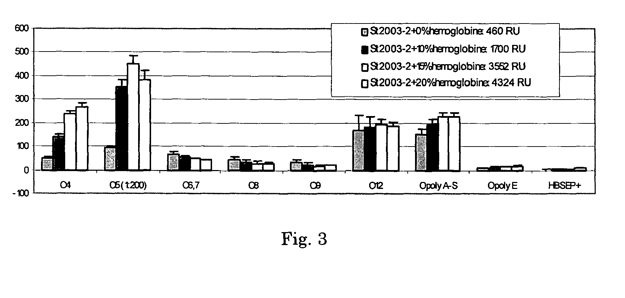

Immobilization levels and expected reactivity of agglutination sera with *S. enteritidis* LPS (batch Se2003.1), *S. goldcoast* (batch Sg2003.2), *S. livingstone* (batch S12003.1), *S. typhimurium* (batch St2003.1) and *S. meleagridis* (Batch Sm2003.1) revealed a correlation with relative amount of Hb added before oxidation (see for example Figure 3). It was also found that for a salmonella serovar-specific LPS type, the optimum Hb concentration was also production batch dependent.
On guidance of maximum response of the expected antigenic profile using a panel of standard and reference control sera and on guidance of low responses from avian SPF reference sera, optimum Hb concentration was determined for each type and for each batch of LPS (Table 21). In the text, oxidized, haemoglobin-containing LPS preparations are further referred to as LPS^{ox}-Hb preparations.
It should be noted that, in any experiment, immobilization level did not correlate with serological responses but correlated with the amount Hb that was added.

**Table 21. Effect of haemoglobin and LPS concentration on the final immobilization level of LPS derived from S. enteritidis (Se) and S. typhimurium (St).**

| **LPS concentration (µg/ml)** | **Se LPS** | | **St LPS** | |
|---|---|---|---|---|
| | **10% (m/m) Hb**^{**a**} | **15% (m/m) Hb** | **10% (m/m) Hb** | **15% (m/m) Hb** |
| 25 | | | | 9197 |
| 62.5^{b} | 2194-2065 | 3173 | | |
| 62.5^{b} | 3400-3000 | | | |
| 62.5^{b} | | | 8414 | |
| 62.5^{b} | 2374 | | | |
| 125 | 3940-3647 | | | |
| 250 | 3370 | | 10645 | |

| | | | | |
|---|---|---|---|---|
| ^{a} Concentration of Hb relative to LPS ^{b} prepared on separate sensor channels. | | | | |

The effect of dilution of Se LPS and St LPS before oxidation in the presence of 10% (m/m) or 15% (m/m) Hb relative to LPS, respectively, was investigated (Table 21). These results did not clearly reveal a correlation between LPS concentration and coupling level.

**Table 22. Relationship between immobilization level of LPS^{ox}-Hb and serologic responses of O antigen antiserum and avian control sera. Fields for which a serological response was expected are shaded in a green color and values within the field are underlined. Control sera were diluted in HBS-EP containing 0.5 M sodium chloride and 0.5% (m/v) carboxymethylated dextran.**

| **LPS batch code** | **immobilization level (RU)** | **control sera (1:20, v/v)** | | | | | |
|---|---|---|---|---|---|---|---|
| | | **O poly A-S** | **C-SPF** | **C-Se** | **C-St** | **C-Spg** | **C-Si** |
| Se2003.1 | 2083 | 253.3 | 24.9 | 1705.9 | 122.5 | 4950.4 | 58.4 |
| | 716 | 95.8 | 14.9 | 681.6 | 56.4 | 1647.4 | 29.1 |
| | 450 | 48.9 | 9.3 | 351.5 | 31.2 | 733.0 | 17.0 |
| | 292 | 17.4 | 10.0 | 89.3 | 15.2 | 195.3 | 10.8 |
| Sg2003.2 | 8709 | 180.0 | 27.2 | 151.1 | 53.9 | 136.8 | 184.4 |
| | 8017 | 181.3 | 29.4 | 149.4 | 50.9 | 137.6 | 186.1 |
| | 6075 | 205.0 | 29.1 | 193.5 | 48.3 | 129.7 | 206.4 |
| | 3793 | 155.4 | 25.7 | 144.5 | 39.1 | 98.9 | 157.9 |
| S12003.1 | 11526 | 83.2 | 51.1 | 69.8 | 76.1 | 115.6 | 3708.2 |
| | 11329 | 90.3 | 54.3 | 74.7 | 76.6 | 121.0 | 3832.5 |
| | 8357 | 97.7 | 54.8 | 77.3 | 66.8 | 111.9 | 4000.1 |
| | 6067 | 88.5 | 51.7 | 73.0 | 61.3 | 104.4 | 3815.1 |
| St2003,1 | 2831 | 175.0 | 32.2 | 466.8 | 607.3 | 1762.3 | 147.4 |
| | 543 | 48.1 | 16.9 | 119.9 | 174.9 | 369.3 | 58.1 |
| | 321 | 26.2 | 11.2 | 64.8 | 97.5 | 179.1 | 36.5 |
| | 217 | 12.2 | 12.1 | 18.9 | 28.8 | 46.0 | 15.8 |

### LPS stability and robustness of immobilization

Reproducibility and repeatability of the immobilization of LPS and the specific response of reference sera were tested. The Se, Sg, Sl and St LPS preparations were oxidized in the presence of their corresponding optimal Hb concentration, desalted, and then stored in solution at 4°C in the dark. Under identical conditions, but accounting with the variability generally observed for immobilization of molecules at a biosensor surface, immobilization levels of oxidized LPS were either comparable in the cases of the Sl (5% RSD) and St (8% RSD) batches or tended to increase in the cases of the Se (13% RSD) and Sg (8% RSD) batches over two months of storage (Table 23 through Table 26). The responses of reference sera were probed on the prepared biosensor chips as well. Inspection of these did not reveal a correlation between immobilization level and specific response. For example, immobilization levels of Se LPS and Sg LPS may tend to increase over time; this increase was not reflected in the response of the binding of serum antibodies to the immobilized antigens. The relative standard deviation varies between 12% and 38%. When considering the first 3 measuring days (day 0, day 7 or 10, and day 14 or 17) the variance is greatly reduced from 3.5% to 23% (results not shown). Tables 27 to 30 summarizes the repeatability of the method at several moments over a 12-months period. At each analysis time point, a fresh aliquot of Hb-fortified LPS was oxidized, immobilized and analysed and thus reflects the sum of variability of several steps.

**Table 23. Response of agglutination and reference anti-sera with LPS^{ox}-Hb Se2003.1 prepared at day 0 and stored at 5-8°C. The LPS preparation was immobilized and tested after oxidation at the days indicated.**

| **Day of analysis** | **immobilization level (RU)** | **Se2003.1 oxidized in presence of 15% Hb** | | | | |
|---|---|---|---|---|---|---|
| | | **O9** | **O12** | **O poly A-S** | **C-Se** | **C-Spg (1:100)** |
| 0 | 2708 | 446 | 302 | 330 | 2597 | 3535 |
| 10 | 3007 | 352 | 258 | 252 | 2205 | 3726 |
| 17 | 3194 | 472 | 320 | 292 | 2520 | 3586 |
| 31 | 3642 | 509 | 356 | 340 | 1486 | 3727 |
| 62 | 3640 | 361 | 223 | 156 | 1676 | 2329 |
| Average | 3238 | 428 | 292 | 274 | 2097 | 3381 |
| St. dev. | 407 | 69 | 52 | 74 | 498 | 594 |
| RSD (%) | 13 | 16 | 18 | 27 | 24 | 18 |

**Table 24. Response of agglutination and reference anti-sera with LPS^{ox}-Hb Sg2003.2 prepared at day 0 and stored at 5-8°C. The LPS preparation was immobilized and tested after oxidation at the days indicated.**

| **Day of analysis** | **immobilization level (RU)** | **Sg2003.2 oxidized in presence of 50% Hb** | | |
|---|---|---|---|---|
| | | **O6,7** | **O8** | **O poly A-S** |
| 0 | 7262 | 668 | 465 | 117 |
| 7 | 9972 | 714 | 531 | 110 |
| 14 | 10597 | 657 | 566 | 111 |
| 20 | 11453 | 857 | 502 | 100 |
| 28 | 11866 | 1085 | 504 | 130 |
| 59 | 12002 | 484 | 364 | 58 |
| Average | 10525 | 744 | 489 | 104 |
| St. dev. | 869 | 226 | 77 | 27 |
| RSD (%) | 8 | 30 | 16 | 26 |

**Table 25. Response of agglutination and reference anti-sera with LPS^{ox}-Hb S12003.1 prepared at day 0 and stored at 5-8°C. The LPS preparation was immobilized and tested after oxidation at the days indicated.**

| **Day of analysis** | **immobilization level (RU)** | **S12003.1 oxidized in presence of 50% Hb** | |
|---|---|---|---|
| | | **O6,7** | **O poly A-S** |
| 0 | 11509 | 318 | 84 |
| 7 | 13581 | 289 | 63 |
| 14 | 14870 | 250 | 54 |
| 20 | 13582 | 475 | 65 |
| 28 | 14976 | 468 | 75 |
| 59 | 14744 | 202 | 31 |
| Average | 13877 | 334 | 62 |
| St.dev. | 707 | 127 | 17 |
| RSD (%) | 5 | 38 | 27 |

**Table 26. Response of agglutination and reference anti-sera with LPS^{ox}-Hb St2003.1 prepared at day 0 and stored at 5-8°C. The LPS preparation was immobilized and tested after oxidation at the days indicated.**

| **Day of analysis** | **immobilization level (RU)** | **St2003.1 oxidized in presence of 15% Hb** | | | | | | |
|---|---|---|---|---|---|---|---|---|
| | | **O4** | **O5 (1:200)** | **O12** | **O poly A-S** | **C-St** | **C-Se** | **C-Spg (1:100)** |
| 0 | 5391 | 594 | 540 | 278 | 362 | 441 | 544 | 1634 |
| 10 | 4432 | 463 | 567 | 242 | 273 | 448 | 356 | 1442 |
| 17 | 4473 | 517 | 578 | 250 | 262 | 362 | 396 | 1294 |
| 31 | 4769 | 559 | 582 | 296 | 326 | 440 | 255 | 1464 |
| 62 | 4996 | 442 | 321 | 209 | 196 | 340 | 297 | 983 |
| Average | 4812 | 515 | 518 | 255 | 284 | 406 | 370 | 1363 |
| St. dev. | 397 | 64 | 111 | 34 | 64 | 51 | 112 | 244 |
| RSD (%) | 8 | 12 | 22 | 13 | 22 | 13 | 30 | 18 |

**Table 27. Responses of freshly oxidized Se LPS (batch Se2003.1) on indicated time points (in months). The LPS was isolated from bacterial cells, fortified with 15% (m/m) Hb, dried and stored at 4-7°C until day of oxidation, immobilization and analysis.**

| **Analysis (month)** | **immobilization level (RU)** | **Se2003.1 oxidized in presence of 15% Hb** | | | | |
|---|---|---|---|---|---|---|
| | | **O9** | **O12** | **O poly A-S** | **C-Se (1:200, v/v)** | **C-Spg (1:100, v/v)** |
| 0 | 2708 | 446 | 302 | 330 | 2597^{a} | 3535^{b} |
| 1^{c} | 1326 | 252 | 165 | 172 | 464 | 1274 |
| 2 | 2380 | 372 | 262 | 230 | 502 | 1478 |
| 3 | 2003 | 398 | 208 | 230 | 676 | 1864 |
| 5 | 2309 | 144 | 207 | 337 | 534 | 1539 |
| 7 | 3487 | 240 | 444 | 547 | 786 | 2368 |
| 9 | 3721 | 215 | 407 | 576 | 705 | 2415 |
| 12 | 1450 | 78 | 145 | 219 | 159 | 1272 |

| | | | | | | |
|---|---|---|---|---|---|---|
| ^{a} serum was diluted 1:50 (v/v) ^{b} serum was diluted 1:100 (v/v) ^{c} LPS was diluted at another volume ratio | | | | | | |

**Table 28. Responses of freshly oxidized Sg LPS (batch Sg2003.2) on indicated time points (in months). The LPS was isolated from bacterial cells, fortified with 50% (m/m) Hb, dried and stored at 4-7°C until day of oxidation, immobilization and analysis.**

| **Analysis (month)** | **immobilization level (RU)** | **Sg2003.2 oxidized in presence of 50% Hb** | | |
|---|---|---|---|---|
| | | **O6,7** | **O8** | **O poly A-S** |
| 0 | 7262 | 668 | 465 | 117 |
| 1 | 7428 | 929 | 451 | 100 |
| 2 | 9023 | 639 | 459 | 91 |
| 3 | 13152 | 474 | 606 | 119 |
| 5 | 8087 | 549 | 446 | 294 |
| 7 | 9724 | 622 | 382 | 286 |
| 9 | 8870 | 692 | 508 | 364 |
| 12 | 7088 | 491 | -* | 281 |

**Table 29. Responses of freshly oxidized S1 LPS (batch S12003.1) on indicated time points (in months). The LPS was isolated from bacterial cells, fortified with 50% (m/m) Hb, dried and stored at 4-7°C until day of oxidation, immobilization and analysis.**

| Analysis (month) | **immobilization level (RU)** | **S12003.1** | |
|---|---|---|---|
| | | **O6,7** | **O poly A-S** |
| 0 | 11509 | 318 | 84 |
| 1 | 11442 | 417 | 62 |
| 2 | 12280 | 287 | 55 |
| 3 | 13152 | 231 | 65 |
| 5 | 11896 | 217 | 159 |
| 7 | 11563 | 271 | 149 |
| 9 | 10882 | 298 | 191 |
| 12 | 10138 | 204 | 131 |

**Table 30. Responses of freshly oxidized St LPS (batch St2003.1) on indicated time points (in months). The LPS was isolated from bacterial cells, fortified with 15% (m/m) Hb, dried and stored at 4-7°C until day of oxidation, immobilization and analysis.**

| **Analysis (month)** | **immobilization level (RU)** | **St2003.1 oxidized in presence of 15% Hb** | | | | | |
|---|---|---|---|---|---|---|---|
| | | **O4** | **O5 (1:200, v/v)** | **O12** | **O poly A-S** | **C-St** | **C-Spg (1:200, v/v)** |
| 0 | 5391 | 594 | 540* | 279 | 362 | 441 | 1634^{a} |
| 1⁶ | 3487* | 339 | 470 | 181 | 207 | 352 | 597 |
| 2 | 4623 | 482 | 325 | 257 | 242 | 411 | 612 |
| 3 | 4079 | 425 | 608 | 187 | 235 | 982 | 624 |
| 5 | 6873 | 359 | 369 | 167 | 302 | 393 | 605 |
| 7 | 5410 | 681 | 734 | 334 | 453 | 559 | 837 |
| 9 | 4008 | 638 | 664 | 278 | 441 | 524 | 824 |

| | | | | | | | |
|---|---|---|---|---|---|---|---|
| ^{a} serum was diluted 1:100 (v/v); ^{b} Following oxidation, LPS-containing solution was diluted twice instead of once. | | | | | | | |

### Description of figures

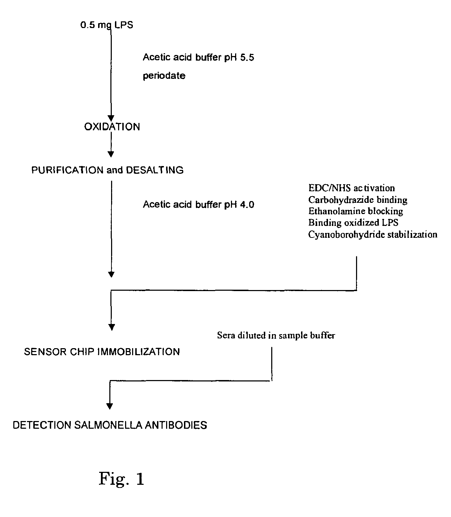

**Figure 1.** Schematic representation of one embodiment of the method according to the invention
**Figure 2.** Schematic outline of the BIA for detection of bacteria using bacteriophages as indicator organisms. Indicator organisms may be cultured overnight or shorter.
**Figure 3.** Reactivity of Hb-fortified, oxidized LPS isolated from *S. typhimurium* (batch St2003.2) with agglutination sera in relative arbitrary biosensor responses (RU). The Hb fortification level of LPS during oxidation isdepicted in the figure. The expected binding of the agglutination sera is listed in Table 3.

### References

Barrow, P., 2000. Serological diagnosis of Salmonella by ELISA and other tests. In: Wray, C., Wray, A. (Eds.), Salmonella in Domestic Animals. CAB International, Oxon, p. 407.
Barrow, P.A., Desmidt, M., Ducatelle, R., Guittet, M., van der Heijden, H.M., Holt, P.S., Huis in't Velt, J.H., McDonough, P., Nagaraja, K.V., Porter, R.E., Proux, K., Sisak, F., Staak, C., Steinbach, G., Thorns, C.J., Wray, C., van Zijderveld, F., 1996. World Health Organisation-supervised interlaboratory comparison of ELISAs for the serological detection of Salmonella enterica serotype enteritidis in chickens. Epidemiol. Infect. 117, 69.
Berends, B.R., van Knapen, F., Mossel, D.A., Burt, S.A., Snijders, J.M., 1998. Impact on human health of Salmonella spp. on pork in The Netherlands and the anticipated effects of some currently proposed control strategies. Int. J. Food Microbiol. 44, 219.
de Vries, N., Zwaagstra, K.A., Huis in't Veld, J.H., van Knapen, F., van Zijderveld, F.G., Kusters, J.G., 1998. Production of monoclonal antibodies specific for the i and 1,2 flagellar antigens of Salmonella typhimurium and characterization of their respective epitopes. Appl. Environ. Microbiol. 64, 5033.
Edel, W., van Leeuwen, W.J., Hoogenboom Verdegaal, A.M., 1993. The annual incidence of salmonellosis in humans in The Netherlands. Tijdschr. Diergeneeskd. 118, 306.
Fratamico, P.M., Strobaugh, T.P., Medina, M.B., Gehring, A.G., 1997. Real-time detection of Escherichia coli O157:H7 using a surface plasmon resonance biosensor. Book of Abstracts, 214th ACS National Meeting, Las Vegas, NV, September 7 - 11, AGFD.
Grimont, P., Grimont, F., Bouvet, P., 2000. Taxonomy of the genus Salmonella. In: Wray, C., Wray, A. (Eds.), Salmonella in Domestic Animals. CAB International, Oxon, p. 1.
Holt, P., 2000. Host susceptibility, resistance and immunity to Salmonella in animals. In: Wray, C., Wray, A. (Eds.), Salmonella in Domestic Animals. CAB International, Oxon, p. 73.
Hoogenboom Verdegaal, A.M., de Jong, J.C., During, M., Hoogenveen, R., Hoekstra, J.A., 1994. Community-based study of the incidence of gastrointestinal diseases in The Netherlands. Epidemiol. Infect. 112, 481.
Ivnitski, D., Abdel-Hamid, I., Atanasov, P., Wilkins, E., 1999. Biosensors for detection of pathogenic bacteria. Biosens. Bioelectron. 14, 599.
Jongerius-Gortemaker, B.G.M. et al., 2002. Surface plasmon resonance (BIACORE) detection of serum antibodies against Salmonella enteritidis and Salmonella typhimurium, Journal of Immunological Methods 266, 33―44
Kamerling JP, Vliegenthart JFG, Carbohydrates. In Clinical Biochemistry, Principles, Methods, Applications. Mass Spectrometry, edited by Lawson AM (Walter de Gruyter, Berlin, 1989), vol. 1, pp. 175-263.
Kretschmann, E., Raether, H., 1968. Radiative decay of nonradiative surface plasmons excited by light. Z. Naturforsch. A 23, 2135.
Liedberg, B., Nylander, C., Lundstroem, I., 1983. Surface plasmon resonance for gas detection and biosensing. Sens. Actuators 4, 299.
Medina, M.B., 1997. SPR biosensor: food science applications. Food Test. Anal. 3, 14.
Medina, M.B., Van Houten, L., Cooke, P.H., Tu, S.I., 1997. Realtime analysis of antibody binding interactions with immobilized E. coli 0157:H7 cells using the BIAcore. Biotechnol. Technol. 11, 173.
Popoff, M.Y. (2001) In: Antigenic formulas of the Salmonella serovars, 8th revision. WHO Collaborating Centre for Reference and Research on Salmonella. Institut Pasteur, Paris, France, pp. 150.
Staub,A.M. (1965) Bacterial lipido-proteino-polysaccharides ('O' somatic antigens): extraction with trichloroacetic acid. In Whistler,R.L. (ed.), Methods in Corbohydrate Chemistry, Volume V. Academic Press, New York, pp. 92-93
Thorns, C.J., Bell, M.M., Sojka, M.G., Nicholas, R.A., 1996. Development and application of enzyme-linked immunosorbent assay for specific detection of Salmonella enteritidis infections in chickens based on antibodies to SEF14 fimbrial antigen. J. Clin. Microbiol. 34, 792.
van Asten, A.J., Zwaagstra, K.A., Baay, M.F., Kusters, J.G., Huis in't Veld, J.H., van der Zeijst, B.A., 1995. Identification of the domain which determines the g,m serotype of the flagellin of Salmonella enteritidis. J. Bacteriol. 177, 1610.
Van Pelt, W., Van de Giessen, A., Van Leeuwen, W., Wannet, W., Henken, A., Evers, E., De Wit, M., Van Duynhoven, Y., 1999. Oorsprong, omvang en kosten van humane salmonellose. Deel 1. Oorsprong van humane salmonellose met betrekking tot varken, rund, kip, ei en overige bronnen. Infectieziekten Bull. 10, 240.
W. van Pelt, W.J.B. Wannet, A.W. van de Giessen, Y.T.H.P. van Duynhoven, 2003. Trends in gastroenteritis in the Netherlands Lowest incidences in 2002 ever; the lull before the storm? Infectieziekten bull. 14, 424.
Wilkons S.G., 1996, Bacterial lipopolysaccharides- Themes and variations. Progress in Lipid Research, volume 35, issue 3, sept, page 283-343
Yamane, Y., Awamura, N., Fujii, H., Ohta, H., Toyota, Y., Otsuki, K., Inoue, T., 2000. Establishment of an enzyme-linked immunosorbent assay with a coated deflagellated Salmonella enteritidis antigen for detection of a specific chicken antibody. Avian Dis. 44, 291.

## Claims

1. A method for immobilisation of a polysaccharide on a carrier, comprising contacting said polysaccharide with an oxidising agent and a protein to obtain a polysaccharide-protein complex and coupling said polysaccharide-protein complex to said carrier.

2. A method according to claim 1, wherein said polysaccharide is derived from a gram-negative bacterium.

3. A method according to claim 1 or 2, wherein said polysaccharide is derived from an enterobacteriaceae.

4. A method according to any one of claims 1 to 3, wherein said polysaccharide is derived from a salmonella (sub)species.

5. A method according to any one of claims 1 to 4, wherein said polysaccharide is a lipopolysaccharide.

6. A method according to any one of claims 1 to 5, wherein said protein is haemoglobin or myoglobin.

7. A method according to any one of claims 1 to 6, wherein said oxidising agent is (sodium)periodate.

8. A method according to any one of claims 1 to 7, further comprising activating the surface of said carrier.

9. A method according to any one of claims 1 to 8, wherein said carrier comprises a glass surface coated with gold.

10. A method according to anyone of claims 1 to 9, wherein said carrier is modified with a carboxymethylated dextran layer.

11. A method according to claim 10, wherein said dextran layer is activated with 1-ethyl-3-(3-dimethylaminopropyl)carbodiimide hydrochloride, N-hydroxysuccinimide and carbohydrazide.

12. A method according to any one of claims 1 to 11, comprising at least two different polysaccharides.

13. A method according to anyone of claims 1 to 12, wherein said carrier is a biosensor chip.

14. A carrier obtained by the method according to any one of claims 1 to 13.

15. A carrier comprising an immobilised polysaccharide-protein complex on its surface.

16. A biosensor comprising a carrier according to claim 14 or 15.

17. A Surface Plasmon Resonance detection system comprising a biosensor according to claim 16.

18. A method for determining the presence of an antibody directed to an antigen of a gram-negative bacteria in a sample, comprising contacting said sample with a carrier according to claim 14 or 15 or a biosensor according to claim 16 and determining whether the carrier has bound any antibody.

19. A method according to claim 18, wherein said sample is blood, blood-derived liquid material, tissue-derived fluids, such as meat drip, milk, egg, fluids from an eye, saliva or faeces.

20. A method for determining the presence of a gram-negative bacterium in a sample, comprising contacting said sample with a predetermined amount of antibodies directed against an antigen of said bacterium and determining the amount of antibodies not bound to said bacterium with a carrier according claim 14 or 15 or a biosensor according to claim 16.

21. A method according to any one of claims 18 to 20, wherein binding to said carrier or said biosensor is determined by Plasmon Surface Resonance.

22. A method according to any one of claims 18 to 21, wherein said sample is obtained from a human or an animal.

23. A method for determining the presence of a gram-negative bacterium in a sample comprising
- contacting said sample with target bacteria-specific, bacteriophages and allowing the bacteriophages to infect said sample
- removing non-bound and/or non-invading bacteriophages resulting in a bacteriophage infected sample
- bringing the bacteriophage infected sample into contact with an indicator organism susceptible for the used bacteriophages
- incubate during at least one bacteriophage multiplication cycle
- recover the bacteriophages to obtain a bacteriophage-containing sample
- analyse said bacteriophage-containing sample with a carrier according to claim 14 or 15 or a biosensor according to claim 16.

24. A method according to claims 23, wherein said sample is obtained from a human, a plant or an animal.
